(19) Europäisches Patentamt
European Patent Office
Office européen des brevets

(11) EP 0 826 054 B1

(12) **EUROPEAN PATENT SPECIFICATION**

(45) Date of publication and mention of the grant of the patent:
**17.12.2003 Bulletin 2003/51**

(21) Application number: **96920281.1**

(22) Date of filing: **16.05.1996**

(51) Int Cl.[7]: **C12N 15/54**, C12N 9/10, C12N 15/62, C12N 1/15 // (C12N1/15, C12R1:66)

(86) International application number:
**PCT/US96/07171**

(87) International publication number:
**WO 9603/6718 (21.11.1996 Gazette 1996/51)**

(54) **EXPRESSION OF GLYCOSYLTRANSFERASE IN ASPERGILLUS**

GLYKOSYLTRANSFERASE EXPRESSION IN ASPERGILLUS

EXPRESSION DE LA GLYCOSYLTRANSFERASE CHEZ ASPERGILLUS

(84) Designated Contracting States:
**AT BE CH DE DK ES FI FR GB GR IE IT LI NL PT SE**

(30) Priority: **18.05.1995 US 443954**

(43) Date of publication of application:
**04.03.1998 Bulletin 1998/10**

(73) Proprietor: **GENENCOR INTERNATIONAL, INC.**
**Palo Alto, California 94304 (US)**

(72) Inventors:
- **KADOMA, Katherine, H.**
  **Palo Alto, CA 94304-1013 (US)**
- **WARD, Michael**
  **Palo Alto, CA 94304-1013 (US)**

(74) Representative: **Kremer, Simon Mark et al**
**Mewburn Ellis,**
**York House,**
**23 Kingsway**
**London WC2B 6HP (GB)**

(56) References cited:

| | |
|---|---|
| **WO-A-86/06097** | **WO-A-90/15860** |
| **WO-A-94/04687** | **WO-A-94/29457** |

- **GENE, AUG 5 1994, 145 (2) P299-303, NETHERLANDS, XP002013110 SCHWIENTEK T ET AL: "Efficient intra- and extracellular production of human beta-1,4-galactosyltransferase in Saccharomyces cerevisiae is mediated by yeast secretion leaders."**
- **J BIOL CHEM, JAN 14 1994, 269 (2) P1394-401, UNITED STATES, XP002013111 KITAGAWA H ET AL: "Cloning of a novel alpha 2,3-sialyltransferase that sialylates glycoprotein and glycolipid carbohydrate groups."**
- **BIOTECHNOLOGY (N Y), MAY 1990, 8 (5) P435-40, UNITED STATES, XP002013112 WARD M ET AL: "Improved production of chymosin in Aspergillus by expression as a glucoamylase-chymosin fusion."**
- **BIOTECHNOLOGY (N Y), OCT 1991, 9 (10) P976-81, UNITED STATES, XP002013113 DUNN-COLEMAN NS ET AL: "Commercial levels of chymosin production by Aspergillus."**
- **BERKA ET AL: 'The development of Aspergillus niger var. awamori as a host for the expression and secretion of heterologous gene products.' BIOCHEMICAL SOCIETY TRANSACTIONS vol. 19, no. 3, August 1991, COLCHESTER, pages 681 - 685**
- **DAVIES R.W.: 'Heterologous Gene Expresion and Protein Secretion in Aspergillus' PROG IND MICROBIOL vol. 29, 1994, pages 527 - 560**
- **MALISSARD ET AL: 'Expression of functional soluble forms of human beta-1,4-galactosyltransferase I, alpha-2,6-sialyltransferase, and alpha-1,3-fucosyltransferase VI in the methylotrophic yeast Pichia pastoris' BIOCHEMICAL AND BIOPHYSICAL RESEARCH COMMUNICATIONS vol. 267, 2000, pages 169 - 173**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 826 054 B1

- **MALISSARD ET AL: 'The yeast expression system for recombinant glycosyltransferases' GLYCOCONJUGATE JOURNAL vol. 16, 1999, pages 125 - 139**

SEQUENCE LISTING

(1) GENERAL INFORMATION:

(i) APPLICANT:

(A) NAME: Kodama, Katherine
(B) STREET: 180 Kimball Way
(C) CITY: So. San Francisco
(D) STATE: CA
(E) COUNTRY: USA
(F) POSTAL CODE (ZIP): 94080
(G) TELEPHONE: (415) 742-7536
(H) TELEFAX: (415) 742-7217

(A) NAME: Ward, Michael
(B) STREET: 180 Kimball Way
(C) CITY: So. San Francisco
(D) STATE: CA
(E) COUNTRY: USA
(F) POSTAL CODE (ZIP): 94080
(G) TELEPHONE: (415) 742-7536
(H) TELEFAX: (415) 742-7217

(ii) TITLE OF INVENTION: Expression of Glycosyltransferase in Aspergillus

(iii) NUMBER OF SEQUENCES: 13

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 1:

ATGGGAAGAC TCGAATTCAC TGATT 25

(2) INFORMATION FOR SEQ ID NO: 2:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 2:

GCCGGACACT GGTCTAGAGA GGCTCAC 27

(2) INFORMATION FOR SEQ ID NO: 3:

(i) SEQUENCE CHARACTERISTICS:

(A) LENGTH: 63 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 3:

TCGACCGCGA CGGTGACCGA CACCTGGCGG AAGCGCCTAC ACTTACTCCA ATGGGAAGAC 60

TCG 63

(2) INFORMATION FOR SEQ ID NO: 4:

(i) SEQUENCE CHARACTERISTICS:

(A) LENGTH: 63 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 4:

AATTCGAGTC TTCCCATTGG AGTAAGTGTA GGCGCTTCCG CCAGGTGTCG GTCACCGTCG 60

CGG 63

(2) INFORMATION FOR SEQ ID NO: 5:

(i) SEQUENCE CHARACTERISTICS:

(A) LENGTH: 57 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 5:

TCGACCGCGA CGGTGACCGA CACCTGGCGG CTACACTTAC TCCAATGGGA AGACTCG 57

(2) INFORMATION FOR SEQ ID NO: 6:

(i) SEQUENCE CHARACTERISTICS:

(A) LENGTH: 57 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 6:

AATTCGAGTC TTCCCATTGG AGTAAGTGTA GCCGCCAGGT GTCGGTCACC GTCGCGG 57

(2) INFORMATION FOR SEQ ID NO: 7:

(i) SEQUENCE CHARACTERISTICS:

(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 7:

CTAGCAAAGCTACA CTTACTCCAA TGGGAAGACT CG 36

(2) INFORMATION FOR SEQ ID NO: 8:

(i) SEQUENCE CHARACTERISTICS:

(A) LENGTH: 35 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 8:

AATTCGAGTC TTACCATTGG AGTAAGTGTA GCTTG 35

(2) INFORMATION FOR SEQ ID NO: 9:

(i) SEQUENCE CHARACTERISTICS:

(A) LENGTH: 38 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 9:

CTAGCAAGCG CCTACACTTA CTCCAATGGG AAGACTCG 38

(2) INFORMATION FOR SEQ ID NO: 10:

(i) SEQUENCE CHARACTERISTICS:

(A) LENGTH: 38 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 10:

AATTCGAGTC TTACCATTGG AGTAAGTGTA GGCGCTTG 38

(2) INFORMATION FOR SEQ ID NO: 11:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 11:

CTAGCTAGCA AGCGCGGATC CCCTAGGCCA 30

(2) INFORMATION FOR SEQ ID NO: 12:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 12:

CCCGGTGACC TCAGGTGAAC CAAGCC 26

(2) INFORMATION FOR SEQ ID NO: 13:

(i) SEQUENCE CHARACTERISTICS:

(A) LENGTH: 24 base pairs
(B) TYPE: nucleic acid
(C) STRANDEDNESS: single

(D) TOPOLOGY: linear

(ii) MOLECULE TYPE: DNA (genomic)

(xi) SEQUENCE DESCRIPTION: SEQ ID NO: 13:

```
CCGAGTATCG TGGCTACTGG CGGC                                            24
```

## Description

### Technical Field of the invention

The present invention is directed to increased secretion of desired polypeptides (particularly glycosyltransferases) from filamentous fungi (particularly *Aspergillus*). The invention discloses DNA sequences, vectors, fusion polypeptides, and processes for obtaining enhanced production and secretion levels of the desired polypeptide. More particularly, the invention discloses DNA sequences, vectors, fusion polypeptides and processes for the increased secretion of sialyltransferase, galactosyltransferase and fucosyltransferase in *Aspergillus*, preferably *A. niger* var. *awamori*.

### Background of the Invention

One of the earlier successes of recombinant DNA technology involved the intracellular expression of the A and B chains of insulin in bacteria as carboxyl fusions to β-galactosidase. Goeddel, D.V. et al. (1979) Proc. Natl. Acad. Sci. USA **76**:106-110; Johnson, I.S. (1983) Science **219**:632-637. Since then, numerous examples have been described for the expression of fusion polypeptides composing, in part, a heterologous polypeptide. Marston, F.A.O. (1986) Biochem. J. **240**:1-12 summarizes the production of heterologous polypeptides in *E. coli*. As described therein, a number of heterologous polypeptides have been expressed intracellularly as fusion polypeptide in *E. coli*. In addition, heterologous polypeptides have reportedly been secreted into the periplasmic space of such microbes by fusing the heterologous polypeptide with a signal sequence. In some cases, the heterologous polypeptide was secreted from *E. coli* into the culture medium when expressed with a signal sequence of bacterial origin. In those cases where a heterologous protein has been expressed as a fusion with the entire native protein of the host bacteria, the rationale was primarily to increase stability or ease the purification of the fusion polypeptide.

For example, Scholtissek, S. et al. (1988) Gene **62**:55-64, report the expression in *E. coli* of a triprotein consisting of bacterial β-galactosidase, a collagenase recognition site and the single stranded DNA binding protein from *E. coli*. The β-galactosidase portion of this fusion polypeptide reportedly was used to purify the fusion polypeptide from a crude cell lysate by affinity chromatography on APTG-Sepharose. The single stranded DNA binding protein *from E. coli* was thereafter isolated from the fusion polypeptide by reacting the collagenase recognition site with collagenase. Similarly, Smith, D.B. et al. (1988) Gene **67**:31-40 report the bacterial expression of a vector encoding a fusion polypeptide consisting of glutathione S-transferase fused at its C-terminus with a recognition site for blood coagulation factor $X_a$ which itself is fused to either of two heterologous polypeptides corresponding to different antigens of *P. falciparum*. In another example, Guan, C. et al. (1988) Gene **67**:21-30 report the expression and purification of fusion polypeptides consisting of maltose binding protein fused either to β-galactosidase or *PstI* endonuclease, and a fusion protein consisting of the bacterial *phoA* signal, maltose binding protein and *phoA* protein. In the former cases, the fusion polypeptides were extracted from crude bacterial lysates by affinity chromatography on cross-linked amylose whereas, in the latter, the fusion protein was obtained from the periplasmic space after spheroplast formation and affinity chromatography on cross-linked amylose.

The expression of fusion polypeptides in yeast has also been reported. For example, Cousens, LS. et al. (1987) Gene **61**:265-275 describe a fusion polypeptide consisting of a human superoxide dismutase-human proinsulin fusion protein with a methionine residue at the junction of the two proteins. Superoxide dismutase is an intracellular protein and the fusion polypeptide was reportedly expressed as an insoluble inclusion body within the yeast expression host with incorrect disulfide bonds. After sulfitollysis proinsulin was reportedly purified, renatured and processed to yield insulin after cleavage of the methionine residue with cyanogen bromide.

United States Patent No. 4,751,180 to Cousens et al. states that a polypeptide of interest may be obtained in high yield from an expression host, such as yeast, when the polypeptide of interest is expressed as a completely heterologous fusion polypeptide. One of the heterologous polypeptides is produced in high yield in the expression host typically in amounts greater than five percent of the total protein produced by the host. The only high yield heterologous polypeptide disclosed, however, is that of the intracellular protein human superoxide dismutase which is fused to either proinsulin or IgF-2. The specification also states that a secretory leader and processing signal may be included as part of the fused polypeptide. No example is provided which indicates that secretion would be obtained and, if obtained, would be at levels higher than that which have been obtained using a fusion construction which detected the high yield heterologous protein in a fusion consisting of a secretory leader sequence fused to only the polypeptide of interest, e. g., proinsulin or insulin-like growth factor (IgF-2).

Heterologous gene expression has also been reported in filamentous fungi. For example, Christensen, T. et al. (1988) Bio/Technology **6**:1419-1422 have reported an expression vector utilizing the α-amylase promotor from *A. oryzae* to express the prepro form of aspartyl proteinase from the filamentous fungus *Rhizomuchor miehei*. When expressed in *A. oryzae*, aspartyl proteinase was obtained from the culture medium. Gwynne, D.I. et al. (1987) Bio/Technology **5**:713-719 report the expression and secretion of human interferon and bacterial endoglucanase from filamentous fungi by expressing these genes with either a fungal glucoamylase signal or a synthetic consensus signal sequence.

US Patent 5,364,770 and EP Patent 0 215 594, teach the expression and secretion of various heterologous polypeptides, such as chymosin, in filamentous fungi, and particularly *Aspergillus*.

Upshall, A. et al. (1987) Bio/Technology **5**:1301-1304 report the expression and secretion of human tissue plasminogen activator by expressing the gene encoding the pre-form of t-PA in a filamentous fungus. Further, Tumbull, I.F. et al. (1989) Bio/Technology **7**:169-174 report an attempt to express and secrete bacterial enterotoxin subunit B from filamentous fungi. No secreted material, however, was detected.

Bovine prochymosin has reportedly been expressed in *Escherichia coli*, the yeasts *Saccharomyces cerevisiae* and *Yarrowia lipolytica*, and in filamentous fungi by the inventor in *Aspergillus* species. In *E. coli* prochymosin, with the first four amino acid residues replaced by an amino-terminal fragment of the *trpE* gene, has reportedly been produced under the control of the *trp* promoter (Nishimori, K. et al. (1984) Gene **29**:41-49). The fusion protein accumulated as inclusion bodies in the cytoplasm but after appropriate extraction conditions could be activated to yield mature chymosin.

Moir et al. (1985) (in Developments in Industrial Microbiology, Vol. 26, Underkofler, L.A. (ed.), Society for Industrial Microbiology, Arlington, VA, USA) described intracellular production of prochymosin in *S. cerevisiae*. The protein was synthesized with various segments of phosphoglycerate kinase, triosephosphate isomerase or galactokinase attached to the amino terminus, allowing increased production compared to direct expression from the same promoters. It was suggested that the increase in production was due to more efficient translation of the mRNA. Moir et al. also reported secretion of prochymosin from *S. cerevisiae*, in the form of a fusion with the first few residues of invertase or alpha factor. The extracellular prochymosin was activated at low pH to give mature chymosin despite the additional amino acids on the prosequence. Similarly, activatable prochymosin was secreted from the yeast *Y. lipolytica* with either 14 or 90 residues of native alkaline extracellular protease attached to the amino terminus (Franke, A.E. et al. (1988) in Developments in Industrial Microbiology, Vol. 29, Pierce, G. (ed.), Society for industrial Microbiology, Arlington, VA, USA). In this report, no more than about 20% of the amino terminus of the protease was used to generate the fusion polypeptides and no apparent advantage accrued from expression as fusion polypeptides. Active calf chymosin has also been produced in the filamentous fungus *Trichoderma reesei* (Harkki, A. et al. (1989) Bio/Technology **7**:596-603. The cellobiohydrolase I gene (*cbhl*) promoter and terminator regions were employed and four different constructions were made employing different signal sequences fused to prochymosin cDNA. Either the chymosin signal sequence, *cbhl* signal sequence, a hybrid *cbhl*/chymosin signal sequence or the *cbhl* signal sequence plus 20 amino acids of mature *cbhl* were fused to the amino terminus of prochymosin. Slightly better production was obtained from the latter construction although insufficient numbers of transformants were examined to confirm this. Secretion was inefficient with approximately 66% of the chymosin-derived material remaining within the cell of transformants regardless of the type of vector construction used.

The *glaA* gene encodes glucoamylase which is highly expressed in many strains of *Aspergillus niger* and *Aspergillus awamori*. The promoter and secretion signal sequence of the *glaA* gene have been used to express heterologous genes in *Aspergilli* including bovine chymosin in *Aspergillus nidulans* and *A. awamori* as previously described by Cullen, D. et al. (1987) Bio/Technology **5**:713-719. In the latter experiments, a variety of constructs were made, incorporating prochymosin cDNA, either the glucoamylase or the chymosin secretion signal and, in one case, the first 11 codons of mature glucoamylase. Maximum yields of secreted chymosin obtained from *A. awamori* were below 15 mg/L in 50 ml shake flask cultures and were obtained using the chymosin signal sequence encoded by pGRG3. These previous studies indicated that integrated plasmid copy number did not correlate with chymosin yields, abundant polyadenylated chymosin mRNA was produced, and intracellular levels of chymosin were high in some transformants regardless of the source of secretion signal. It was inferred that transcription was not a limiting factor in chymosin production but that secretion may have been inefficient. It was also evident that the addition of a small amino-terminal segment (11 amino acids) of glucoamylase to the propeptide of prochymosin did not prevent activation to mature chymosin. The amount of extracellular chymosin obtained with the first eleven codons of glucoamylase, however, was substantially less than that obtained when the glucoamylase signal was used alone.

USSN 08/318,491 and EP Application WO90/15860, disclose the enhanced secretion of desired polypeptides by and from filamentous fungi including fusion DNA sequences, expression vectors containing such DNA sequences, transformed filamentous fungi, fusion polypeptides and processes for expressing and secreting high levels of such desired polypeptides. Specifically described is the enhanced secretion of chymosin from filamentous fungi including fusion DNA sequences, vectors containing such DNA sequences, transformed filamentous fungi, fusion chymosin polypeptides and processes for expressing and secreting high levels of chymosin.

Despite these publications regarding the expression and secretion of various polypeptides from various filamentous fungi, including the production of desired polypeptides in filamentous fungi as fusions, there is a need to provide enhanced production of certain desired polypeptides, particularly glycosyltransferase enzymes, from *Aspergil-*

*lus*, and particularly *A. niger*. The tetrasaccharide sialyl Lewis x serves as a ligand for a family of cell adhesion molecules, known as selectins, which mediate the recruitment of neutrophils and other leucocytes to sites of inflammation and tissue injury. An oligosaccharide mimicking sialyl Lewis x ((Cylexin® to be commercialized by Cytel, San Diego, CA) is being synthesized by combined chemical and enzymatic means for testing as a treatment for inflammatory diseases. Enzymatic synthesis involves using glycosyltransferases such as α-2,3-(N) sialyltransferase (ST), β-(1,4) galactosyl transferase (GT) and α-(1,3) fucosyltransferase (FT) in conjunction with other enzymes that regenerate the donor sugar nucleotide substrate. Commercial scale production of the enzymes GT, FT and ST is difficult and, therefore, the present inventors have proposed to produce such transferases as fusions in an *Aspergillus* system. Using α-2,3-(N) sialyltransferase as a prototype for glycosyltransferase production, α-2,3-(N) sialyltransferase activity was tested in a variety of systems, including baculovirus, mammalian cells and filamentous fungus, using multiple vector constructs. The filamentous fungus *Aspergillus niger* var. *awamori*, advantageously, was found to produce consistent, high levels of α-2,3-(N) sialyltransferase that facilitates cost effective synthesis of the complex carbohydrates.

Accordingly, an object of the present invention is to provide for the expression and enhanced secretion of certain glycosyltransferases in *Aspergillus*, including fusion DNA sequences, expression vectors, transformed host cells, fusion transferases and processes for expressing and secreting high levels of such transferases. Glycosyltransferases of eukaryotes, which are involved in the glycosylation of proteins, are normally located within the secretory apparatus of the cell. They typically possess a transmembrane anchor sequence which serves to localize them on a membrane. The transmembrane coding sequence is preferably deleted from the glycosyltransferase encoding DNA sequence for expression in *Aspergillus*.

The references discussed above are provided solely for their disclosure prior to the filing date of the instant case. Nothing herein is to be construed as an admission that the inventors are not entitled to antedate such disclosure by virtue of prior invention or priority based on earlier filed applications.

## Summary of the Invention

In accordance with the above objects, the invention includes novel fusion DNA sequences comprising from the 5' terminus: 1) a DNA sequence encoding a signal peptide functional as a secretory sequence in *Aspergillus*; 2) a DNA sequence encoding a secreted polypeptide or portion thereof which is normally secreted from a filamentous fungus, and particularly from *Aspergillus*; 3) optionally a DNA sequence encoding a cleavable linker polypeptide; and 4) a DNA sequence encoding a desired glycosyltransferase polypeptide. When the fusion DNA sequence is expressed in *Aspergillus*, increased secretion of the desired polypeptide is obtained as compared to that which is obtained when the desired polypeptide is expressed from DNA sequences encoding a fusion polypeptide which does not contain the second polypeptide normally secreted from an *Aspergillus* host or as compared to the production of such glycosyltransferase in other expression systems such as baculovirus.

The invention also includes expression vectors containing the above fusion DNA sequence and *Aspergillus* strains transformed with such expression vectors. The invention also includes the fusion polypeptide encoded by such fusion DNA sequences.

Further, the invention includes a process for producing a desired glycosyltransferase polypeptide comprising transforming a host *Aspergillus* strain with the above described expression vector and culturing the host strain to secrete the desired polypeptide into the culture medium.

## Brief Description of the Drawings

Figure 1 shows a diagram of the 3-D organization of *Aspergillus* glucoamylase.

Figures 2 A-E show the construction of pGAST and pGASTK, including Seq ID Nos. 1 and 2 in Figure 2A; and Seq ID Nos. 3-6, respectively, in Figure 2B.

Figures 3 A-E show the construction of pGADVST and pGADVSTK, including Seq ID Nos. 7-10, respectively, in Figure 3A.

Figure 4 shows Sheftone N concentration effect.

Figure 5 shows effect of media pH after inoculation.

Figure 6 shows effect of sodium citrate.

Figure 7 shows the map of pGAKHi+, an intermediate vector for FT expression.

Figure 8 shows the construction of pGAKHi+FT.

Figure 9 shows the effect of agitation speed on FT activity in KK17 transformant.

Figure 10 shows the effect of pH on FT activity in KK17 transformant.

Figure 11 shows the construction of pVL1393-FT.

## Detailed Description

The inventors have discovered that desired glycosyltransferase polypeptides can be expressed and secreted at levels higher than that previously obtained from other expression systems such as baculovirus or mammalian cells by expressing the transferase as a fusion of the transferase with a polypeptide which is normally secreted from an *Aspergillus* host system.

Many glycosyltransferases useful in the present invention have been cloned. For example, those listed in the following Table have been cloned and described in the literature.

| Cloned Glycosyltransferases | | |
|---|---|---|
| I. Fucosyltransferases | E.C. Number | Reference |
| α1,2 | 2.4.1.69 | (1) (2) (3) |
| α1,3/4 (FTIII) | - | (4) |
| α1,3 (FTIV) | - | (5) (6) (7) (8) |
| α1,3 (FTV) | - | (9) |
| α1,3 (FTVI) | - | (10)(11) |
| α1,3 (FTVII) | - | (12) |
| II. Galactosyltransferases | | |
| α1,3 | 2.4.1.151 | (13) (14) (15) |
| α1,3 | - | (16) |
| β1,4 | 2.4.1.38 | (17) - (21) |
| III. N-Acetylgalactosaminyltransferases | | |
| α1,3 | - | (22) (23) |
| β1,4 | - | (24)(25) |
| β1,4 (CT antigen) | - | (26) |
| α1,Thr polypeptide | - | (27) (28) |
| IV. N-Acetylglucosaminyltransferases | | |
| β1,2 GnTI | 2.4.1.101 | (29) - (32) |
| β1,2 GnTII | - | (33) |
| β1,4 GnTIII | 2.4.1.144 | (34) (35) |
| β1,6 GnTV | - | (36) (37) |
| β1,6 GnT (O-linked) | - | (38) |
| β1,6 GnT | - | (39) |
| GPT | - | (40) - (43) |
| Hyaluronan synthetase | - | (44) |
| V. Sialyltransferases | | |
| α2,6 (N) | 2.4.99.1 | (45) - (50) |
| α2,6 (O) | 2.4.99.3 | (51) |
| α2,6 (O) | - | (52) |
| α2,3 (N) | 2.4.99.6 | (53) (54) |
| α2,3 (O) | 2.4.99.4 | (55) (56) |

(continued)

| Cloned Glycosyltransferases | | |
|---|---|---|
| V. Sialyltransferases | | |
| α2,3(O) | - | (57) |
| a2,3 (O/N) | - | (58) (59) |
| α2,8 | - | (60) (61) |
| STX | - | (62) |
| VI. Glucosyltransferases | | |
| Alg8 | - | (63) |
| Alg5 | - | (64) |
| VII. Mannosyltransferases | | |
| α1,2 | - | (65) |
| α1,3 (Alg2) | - | (66) |
| β1,4 (Alg1) | - | (67) |
| Dpm1 | - | (68) |
| Och1 | - | (69) |
| Pmt | - | (70) |

**References**

(1) Emst et al. (1989) J. Biol. Chem. **264**:3436-3447
(2) Larsen et al. (1990) Proc. Natl. Acad. Sci. **87**:6684-6678
(3) Rajan et al. (1989) J. Biol. Chem. **264**:11158-11167
(4) Kukowska-Latallo et al. (1990) Genes Dev. **4**:1288-1303
(5) Lowe et al. (1990) Cell **63**:475-484
(6) Goelz et al. (1990) Cell **63**:1349-1376
(7) Lowe et al. (1991) J. Biol. Chem. **266**:17467-17477
(8) Kumar et al. (1991) J. Biol. Chem. **266**-21777-21783
(9) Weston et al. (1992) J. Biol. Chem. **267**:4152-4160
(10) Weston et al. (1992) J. Biol. Chem. **267**:24575-24584
(11) Koszdin and Bowen (1992) BBRC **187**:152-157
(12) Natsuka et al. (1994) J. Biol. Chem. **269**:
(13) Larsen et al. (1989) Proc. Natl. Acad. Sci. **86**:8227-8231 (murine)
(14) Joziasse et al. (1989) J. Biol. Chem. **264**:14290-14297 (bovine)
(15) Dabkowski et al. (1993) Transplant. Proc. **25**:2921
(16) Yamamoto et al. (1990) Nature **345**:229-233
(17) Shaper et al. (1988) J. Biol. Chem. **263**:10420-10428
(18) Narimatsu et al. (1986) Proc. Natl. Acad. Sci. **83**:4720-4724
(19) Masri et al. (1988) BBRC **157**:657-663 (human)
(20) D'Agostaro et al. (1989) Eur. J. Biochem. **183**:211-217 (bovine)
(21) Russo et al. (1990) J. Biol. Chem. **265**:3324-3331
(22) Yamamoto et al. (1990) Nature **345**:229-233
(23) Yamamoto et al. (1990) J. Biol. Chem. **264**:1146-1151
(24) Nagata et al. (1992) J. Biol. Chem. **267**:12082-12089
(25) Furakawa et al. (1993) Trends Glycoscience Glycotech. 5:171-179
(26) Smith and Lowe (1994) J. Biol. Chem. **269**:15162-15171
(27) Hagen et al. (1993) J. Biol. Chem. **268**:18960-18965
(28) Homa et al. (1993) J. Biol. Chem. **268**:12609-12616
(29) Pownall et al. (1992) Genomics **12**:699-704 (mouse)
(30) Kumar et al. (1990) PNAS **87**:9948-9952 (human)
(31) Hull et al. (1991) BBRC **176**:608-615 (human)

(32) Sarkar et al. (1991) Proc. Natl. Acad. Sci. **88**:234-238 (rabbit)
(33) Schaeter, unpublished
(34) Nishikawa et al. (1992) J. Biol. Chem. **267**:18199-18204
(35) Ihara et al. (1993) J. Biochem. **113**:692-698
(36) Bierhuizen et al. (1993) Genes Dev. **7**:468-478
(37) Shoreiban et al. (1993) J. Biol. Chem. **268**:15381-15385
(38) Bierhuizen and Fukuda (1992) PNAS **89**:9326-9330
(39) Fukuda, unpublished
(40) Datta and Lehrman (1993) J. Biol. Chem. **268**:12663-12668
(41) Zhu and Lehrman 91990) J. Biol. Chem. **265**:14250-14255
(42) Scocca and Krag (1990) J. Biol. Chem. **165**:20621-20626
(43) Rajput et al. (1992) Biochem. J. **285**:985-992
(44) DeAngelis et al. (1993) J. Biol. Chem. **268**:19181-19184
(45) Weinstein et al. (1987) J. Biol. Chem. **262**:17735-17743
(46) Colley et al. (1989) J. Biol. Chem. 264:17619-17622
(47) Grundmann et al. (1990) Nucl. Acids Res. **18**:667
(48) Bast et al. (1992) J. Cell Biol. **116**:423-435
(49) Hamamoto et al. (1993) Bioorg. Medic. Chem.
(50) Kurosawa et al. (1994) Eur. J. Biochem. **219**:375-381
(51) Kurosawa et al. (1994) J. Biol. Chem. **269**:1402-1409
(52) Kurosawa et al. (1994) submitted
(53) Wen et al. (1992) J. Biol. Chem. **267**:21011-21019
(54) Kitagawa and Paulson (1993) Biochem. Biophs. Res. Comm. **194**:375-382
(55) Gillespie et al. (1992) J. Biol. Chem. **267**:21004-21010
(56) Lee et al. (1993) Eur. J. Biochem. **216**:377-385
(57) Lee et al., J. Biol. Chem. **269**:10028-10033
(58) Kitagawa and Paulson (1994) J. Biol. Chem. **269**:1394-1401
(59) Sasaki et al. (1993) J. Biol. Chem. **268**:22782-22787
(60) Weisgerber et al. (1991) Glycobiology **1**:357-365
(61) J. Biol. Chem. **268**:11504-11507 (rat)
(62) Livingston and Paulson (1993) J. Biol. Chem. **268**:11504-11507
(63) Stagljov et al. (1994) PNAS **91**:5977
(64) Heesen et al. (1994) Eur. J. Biochem. **224**:71-79
(65) Haeusler and Robbins (1992) Glycobiology **2**:77-84
(66) Jackson et al. (1992) Glycobiology **3**:357-364
(67) Albright et al. (1989) J. Biol. Chem. **263**:7042
(68) Orlean et al. (1988) J. Biol. Chem. **263**:17499-17507
(69) Nakayama et al. (1992) EMBO **11**:2511-2519
(70) Strahl-Bahlsinger et al.

By using the fusion DNA constructions in *Aspergillus* as described herein, a dramatic increase in extracellular glycosyltransferase has been obtained versus expression in other systems. In some cases expression levels are approximately 10 fold in excess of that obtained in other expression systems.

The increased secretion levels of glycosyltransferase are the result of expressing the transferase as a fusion polypeptide with a polypeptide normally secreted by a filamentous fungus. In the preferred embodiments, glucoamylase from *A. niger* var. *awamori* encoded by the *glaA* gene, including the secretory signal sequence of glucoamylase, is fused to the amino terminus of the transferase from which the transmembrane anchor region has been deleted. The presence of the glucoamylase signal sequence and all or part of mature glucoamylase peptide sequences facilitate the enhanced secretion of the fusion polypeptide into the culture medium.

As used herein, a "fusion DNA sequence" comprises from 5' to 3' DNA encoding: a signal peptide; a secreted polypeptide or a portion thereof; optionally a cleavable linker polypeptide; and a desired glycosyltransferase. Specifically, the fusion DNA comprises DNA encoding a signal peptide functional as a secretory sequence in *Aspergillus*. Such signal sequences include those from glucoamylase, α-amylase and aspartyl proteases from *Aspergillus awamori, Aspergillus niger, Aspergillus oryzae*, signal sequences from cellobiohydrolase I, cellobiohydrolase II, endoglucanase I, endoglucanase II from *Trichoderma*, signal sequences from glucoamylase from *Neurospora* and *Humicola*, as well as signal sequences from eukaryotes, including the signal sequence from bovine chymosin, human tissue plasminogen activator, human interferon and synthetic consensus eukaryotic signal sequences such as that described by Gwynne (1987) *supra*. Particularly preferred signal sequences are those derived from polypeptides secreted by the expression

host used to express and secrete the fusion polypeptide. For example, the signal sequence from glucoamylase from *Aspergillus awamori* is preferred when expressing and secreting a fusion polypeptide from *Aspergillus awamori*.

In addition, the fusion DNA sequence comprises DNA encoding "secreted polypeptides" normally expressed from filamentous fungi. Such secreted polypeptides include but are not limited to glucoamylase, α-amylase and aspartyl proteases from *Aspergillus awamori, Aspergillus niger*, and *Aspergillus oryzae*, cellobiohydrolase I, cellobiohydrolase II, endoglucanase I and endoglucanase II from *Trichoderma* and glucoamylase from *Neurospora* species and *Humicola species*. As with the signal sequences, preferred secreted polypeptides are those which are naturally secreted by the *Aspergillus* expression host Thus, for example, when using *Aspergillus awamori*, preferred secreted polypeptides are glucoamylase and α-amylase from *Aspergillus awamori*, most preferably glucoamylase.

Fusion DNA sequences may further comprise DNA sequences encoding a deavable linker polypeptide. Such sequences include those which encode the prosequence of bovine chymosin, the prosequence of subtilisin, prosequences of retroviral proteases, including human immunodeficiency virus protease and DNA sequences encoding amino add sequences recognized and cleaved by trypsin, factor $X_a$ collagenase, dostripin, subtilisin, chymosin, fungal KEX2 protease and the like. See, e.g., Marston, F.A.O. (1986) Biol. Chem. J. **240**:1-12. Such linker sequences may also encode the amino acid methionine which may be selectively cleaved by cyanogen bromide. It should be understood that the linker sequence need only encode that amino add sequence which is necessary to be recognized by a particular enzyme or chemical agent to bring about deavage of the fusion polypeptide. Thus, the entire prosequence of, for example, chymosin or subtilisin need not be used. Rather, only that portion of the prosequence which is necessary for recognition and cleavage by the appropriate enzyme is required. For example, the linker may comprise a recognition sequence (Lys-Arg) for the key protease which sequence may be inserted at the fusion junction as in pGASTK.

Furthermore, the fusion DNA sequence comprises a "desired glycosyltransferase polypeptide." Such desired polypeptides include any glycosyltransferase as provided in the Table above. Preferred glycosyltransferases useful in the present invention include α-(2,3) sialyltransferase; β-(1,4) galactosyltransferase and α-(1,3) fucosyltransferase.

The above-defined DNA sequences encoding the corresponding amino acid sequences are combined to form a "fusion DNA sequence." Such fusion DNA sequences are assembled in proper reading frame from the 5' terminus to 3' terminus in the order presented above. As so assembled, the DNA sequence will encode a "fusion polypeptide" encoding from its amino terminus a signal peptide functional as a secretory sequence in *Aspergillus*, a secreted polypeptide or portion thereof normally secreted from *Aspergillus*, a deavable linker polypeptide and a glycosyltransferase.

As indicated, the DNA encoding a signal peptide is functional as a secretory signal in an *Aspergillus* regardless of whether the signal peptide is from an *Aspergillus* or from some other source such as the chymosin signal from bovine species. The signal sequences may be derived from a secreted polypeptide from a particular species of *Aspergillus*. As indicated, all or part of the mature sequence of the secreted polypeptide is used in the construction of the fusion DNA sequences. In a preferred embodiment of the present invention, the secreted polypeptide is glucoamylase from *A. niger* or *A. niger* var. *awamori*.

*Aspergillus niger* and *Aspergillus niger* var. *awamori* (*A. awamori*) glucoamylases have identical amino acid sequences. The glucoamylase is initially synthesized as preproglucoamylase. The pre and pro regions are removed during the secretion process so that mature glucoamylase is released to the external medium. Two forms of mature glucoamylase are recognized in culture supematants, GAI which is the full-length form (amino acid residues 1-616) and GAII which is a natural proteolytic fragment comprising amino acid residues 1-512. GAI is known to fold as two separate domains joined by an extended linker region. The two domains are the 471 residue catalytic domain (amino acids 1-471) and the 108 residue starch binding domain (amino acids 509-616), the linker region being 36 residues in length (amino acids 472-508). GAII lacks the starch binding domain. These details of glucoamylase structure are reviewed by Libby et al. (Protein Engineering **7**:1109-1114, 1994) and are shown in diagrammatic form in Fig. 1.

Desired polypeptides have previously been produced in filamentous fungi as fusions with a naturally secreted fungal polypeptide. In each case where a comparison has been made between production of desired polypeptide as a fusion with a naturally secreted fungal polypeptide versus production of the desired polypeptide alone (i.e., not fused to another polypeptide), a significant increase in yields of secreted desired polypeptide resulted from production as a fusion protein. Bovine chymosin and porcine pancreatic prophospholipase $A_2$ have both been produced in *A. niger* or *A. niger* var. *awamori* as fusions to full-length GAI (USSN 08/318,494; Ward et al., Bio/Technology **8**:435-440,1990; Roberts et al., Gene **122**:155-161, 1992). Human interleukin 6 (hIL6) has been produced in *A. nidulans* as a fusion to full-length *A. niger* GAI (Contreras et al., Biotechnology **9**:378-381, 1991).

Fusions of desired polypeptides with truncated forms of glucoamylase have also been produced. Unpublished work by the inventors has shown that fusion of bovine prochymosin to glucoamylase residues 1-501 leads to similar yields of chymosin as fusion of prochymosin to full-length glucoamylase. Hen egg white lysozyme (Jeenes et al., FEMS Microbiol. Lett. **107**:267-272, 1993) and human lactoferrin (Ward et al., Bio/Technology **13**:498-503, 1995) have been produced in *A. niger* as fusions to residues 1-498 of glucoamylase and hIL6 has been produced in *A. niger* as a fusion to glucoamylase residues 1-514 (Broekhuijsen et al., J. Biotechnol. **31**:135-145, 1993). In some of the above experiments (Contreras et al., 1991; Broekhuijsen et al., 1993; Ward et al., 1995) a KEX2 protease recognition site (Lys, Arg) has been inserted between glucoamylase and the desired polypeptide to allow *in vivo* release of the desired polypeptide from the fusion protein as a result of the action of a native *Aspergillus* KEX2-like protease.

Additionally, bovine chymosin has been produced in *A. niger* as a fusion with full-length native alpha-amylase (Korman et al., Curr. Genet. **17**:203-212, 1990) and in *A. oryzae* as a fusion with truncated forms of *A. oryzae* glucoamylase (either residues 1-603 or 1-511; Tsuchiya et al., Biosci. Biotech. Biochem. **58**:895-899, 1994).

*Trichoderma reesei* produces several cellulase enzymes, including cellobiohydrolase I (CBHI), which are folded into two separate domains (catalytic and binding domains) separated by an extended linker region. Foreign polypeptides have been secreted in *T. reesei* as fusions with the catalytic domain plus linker region of CBHI (Nyyssonen et al., Bio/Technology **11**:591-595, 1993).

Without intending to be limited to a particular theory, applicants suggest that the amount of glucoamylase or other naturally secreted fungal polypeptide which is needed to enhance the production of a desired polypeptide can be surmised based on the known 3-D structure of glucoamylase. In the case of bovine chymosin production by *A. niger* var. *awamori* it has been shown that fusion of prochymosin following the 11th amino acid of mature glucoamylase provided no benefit compared to production of preprochymosin (US Patent 5,364,770). The inventors have shown (USSN 08/318,494) that fusion of prochymosin onto the C-terminus of preproglucoamylase up to the 297th amino acid of mature glucoamylase plus a repeat of amino acids 1-11 of mature glucoamylase yielded no secreted chymosin in *A. niger* var. *awamori*. In the latter case it is unlikely that the portion (approximately 63%) of the glucoamylase catalytic domain present in the fusion protein was able to fold correctly so that an aberrant, mis-folded and/or unstable fusion protein may have been produced which could not be secreted by the cell. The inability of the partial catalytic domain to fold correctly may have interfered with the folding of the attached chymosin. Thus, it is likely that sufficient residues of a domain of the naturally secreted polypeptide must be present to allow it to fold in its normal configuration independently of the desired polypeptide to which it is attached. Evans et al. (Gene **91**:131-134, 1990) showed that a form of *A. niger* glucoamylase truncated at its C-terminus back to amino acid 460 in the catalytic domain was not efficiently secreted by yeast However, the relevance of this to secretion in *Aspergillus* is not known.

A linker region between the desired polypeptide and the domain of the naturally secreted fungal polypeptide may be beneficial in allowing the two polypeptides to fold independently. However, it may be possible to dispense with the linker region altogether. In the examples above where a foreign polypeptide is fused to the C-terminus of full-length glucoamylase or alpha-amylase, there is no linker separating the desired polypeptide from the C-terminus of the fungal polypeptide. Linkers other than the glucoamylase or CBHI linkers mentioned above could be used to connect a domain of a naturally secreted fungal polypeptide and the desired polypeptide. The salient features of a linker region are that it forms an extended, semi-rigid spacer between independently folded domains. Such linker regions are found in several proteins, especially hydrolases such as bacterial and fungal cellulases and hemicellulases (reviewed in Libby et al. 1994).

Based on the above, either the full length secreted polypeptide or a functional portion thereof may be employed in the present invention. As used herein a "portion" of a secreted polypeptide is defined functionally as that portion of a secreted polypeptide which when combined with the other components of the fusion polypeptide defined herein results in increased secretion of the desired polypeptide as compared to the level of desired polypeptide secreted when an expression vector is used which does not utilize the secreted polypeptide. Generally, such portions of the secreted polypeptide comprise greater than 50% of the secreted polypeptide.

Specific expression hosts include *A. nidulans* (Yelton, M. et al. (1984) Proc. Natl. Acad. Sci. USA **81**: 1470-1474; Mullaney, E.J. et al. (1985) Mol. Gen. Genet. **199**:37-45; John, M.A. and Peberdy, J.F. (1984) Enzyme Microb. Technol. **6**:386-389; Tilburn et al. (1982) Gene **26**:205-221; Ballance, D.J. et al. (1983) Biochem. Biophys. Res. Comm. **112**:284-289; Johnston, I.L. et al. (1985) EMBO **4**:1307-1311); *A. niger* (Kelly, J.M. and Hynes M. (1985) EMBO **4**:475-479); *A. awamori*, e.g., NRRL 3112, ATCC 22342, ATCC 44733, ATCC 14331 and strain UVK 143f; *A. oryzae*, e.g., ATCC 11490; *N. crassa* (Case, M.E. et al. (1979) Proc. Natl. Acad. Sci. USA **76**:5259-5263; Lambowitz US Patent No. 4,486,553; Kinsey, J.A. and Rambosek, J.A. (1984) Molecular and Cellular Biology **4**:117-122; Bull, J.H. and Wooton, J.C. (1984) Nature **310**:701-704); *Trichoderma reesei*, e.g. NRRL 15709, ATCC 13631, 56764, 56765, 56466, 56767 and *Trichoderma viride*, e.g., ATCC 32098 and 32086. A preferred expression host is *A. niger* var. *awamori* in which the gene encoding the major secreted aspartyl protease has been deleted. The production of this preferred expression host is described in USSN 214,237 filed July 1, 1988.

As used herein, a "promotor sequence" is a DNA sequence which is recognized by the particular *Aspergillus* species for expression purposes. It is operably linked to a DNA sequence encoding the above defined fusion polypeptide. Such linkage comprises positioning of the promoter with respect to the translation initiation codon of the DNA sequence encoding the fusion DNA sequence. The promoter sequence contains transcription and translation control sequences which mediate the expression of the fusion DNA sequence. Examples include the promoter from the *A. awamori* or *A. niger* glucoamylase genes (Nunberg, J.H. et al. (1984) Mol. Cell. Biol. **4**:2306-2315; Boel, E et al. (1984) EMBO **3**:1581-1585), the *Mucor miehei* carboxyl protease gene (EPO Patent 0 215 594), the *Trichoderma reesei*

cellobiohydrolase I gene (Shoemaker, S.P. et al. (1984) European Patent Application No. EP 0137 280 A1), the *A. nidulans trpC* gene (Yelton, M. et al. (1984) Proc. Natl. Acad. Sci. USA **81**:1470-1474; Mullaney, E.J. et al. (1985) Mol. Gen. Genet. **199**:37-45), the *A. nidulans alcA* gene (Lockington, R.A. et al. (1986) Gene **33**:137-149), the *A. nidulans tpiA* gene (McKnight, G.L et al. (1986) Cell **46**:143-147), the *A. nidulans amdS* gene (Hynes, M.J. et al. (1983) Mol. Cell Biol. **3**:1430-1439), and higher eukaryotic promoters such as the SV40 early promoter (Barday, S.L. and Meller, E. (1983) Molecular and Cellular Biology **3**:2117-2130).

Likewise a "terminator sequence" is a DNA sequence which is recognized by the expression host to terminate transcription. It is operably linked to the 3' end of the fusion DNA encoding the fusion polypeptide to be expressed. Examples include the terminator from the *A. nidulans trpC* gene (Yelton, M. et al. (1984) Proc. Natl. Acad. Sci. USA **81**:1470-1474; Mullaney, E.J. et al. (1985) Mol. Gen. Genet. **199**:37-45), the *A. awamori* or *A. niger* glucoamylase genes (Nunberg, J.H. et al. (1984) Mol. Cell. Biol. **4**:2306-253; Boel, E. et al. (1984) EMBO **3**:1581-1585), and the *Mucor miehei* carboxyl protease gene (EPO Patent No. 0 215 594), although any fungal terminator is likely to be functional in the present invention.

A "polyadenylation sequence" is a DNA sequence which when transcribed is recognized by the expression host to add polyadenosine residues to transcribed mRNA. It is operably linked to the 3' end of the fusion DNA encoding the fusion polypeptide to be expressed. Examples include polyadenylation sequences from the *A. nidulans trpC* gene (Yelton, M. et al. (1984) Proc. Nad. Acad. Sci. USA **81**:1470-1474; Mullaney, E.J. et al. (1985) Mol. Gen. Genet. **199**: 37-45), the A. *awamori* or *A. niger* glucoamylase genes (Nunberg, J.H. et al. (1984) Mol. Cell. Biol. **4**:2306-2315; Boel, E. et al. (1984) EMBO **3**:1581-1585), and the *Mucor miehei* carboxyl protease gene (EP Patent 0 215,594). Any fungal polyadenylation sequence, however, is likely to be functional in the present invention.

## Examples

Example 1

### Construction of pGAST and pGASTK

These plasmids were made to express the truncated form of alpha 2,3 sialyltransferase as a fusion of the entire glucoamylase coding region which included the catalytic core, the linker region and the starch binding domain in *Aspergillus niger* var. *awamori*. The truncated form of sialyltransferase begins with leucine, the thirtieth amino acid from the initial methionine of the mature protein sequence. A *Bam*HI/*Hind*III fragment of rat liver alpha 2,3 sialyltransferase which included all of the structural gene and some 5' and 3' flanking ends was subcloned into pUC219. The original clone, pGIR23ST (Ichikawa et al. (1992) J. Am. Chem. Soc. **114**:9283-9298), contained the full length α-2,3 sialyltransferase gene. A *Bam*HI fragment of the gene was provided by Cytel Corp. (San Diego, CA, USA). Single stranded template was made of the resulting plasmid, pUC219:ST. Oligonucleotides were used to introduce a unique *Eco*RI restriction site at the 5' region of sialyltransferase and a unique *Xba*I site at the 3' end of the gene. The *Eco*RI site was located at amino acid number 38 and 39 of the mature protein sequence and the oligonucleotide used was 5' ATGGGAAGACTCGAATTCACTGATT 3' (Seq ID No. 1). An *Xba*I site was introduced 17 bases after the stop codon of ST using the oligonucleotide 5' GCCGGACACTGGTCTAGAGAGGCTCAC 3' (Seq ID No. 2). Site directed mutagenesis as described by Maniatis et al. (in Molecular Cloning, A Laboratory Manual, 1980, Cold Spring Harbor Laboratory, Cold Spring Harbor, New York) was used to introduce these sites into the single stranded template of pUC219: 23ST. The correct clones of pUC219:ST R1/Xba were identified by restriction analysis and verified by DNA sequencing. This plasmid was subsequently digested with *Eco*RI and *Xba*I and the 1.0 kb sialyltransferase fragment was isolated for ligations.

Linker oligonucleotides were designed to: 1) connect the end of the glucoamylase coding region from a *Sal*I site, 31 base pairs from the 3' end of the coding region, to the truncated version of sialyltransferase; 2) include or not include a KEX2 protease cleavage site between the structural genes; and 3) reconstruct the final part of the *glaA* coding region from the *Sal*I site to the end of *glaA* coding region (minus the stop codon), as well as reconstruct the sialyltransferase gene from leucine codon #30 to the introduced *Eco*RI at codons 38 and 39. The KEX2 cleavage site which is recognized by the KEX2 protease is made up of two amino acids, lysine and arginine being the favored pair for cleavage. The pair of complimentary oligonucleotides used with the KEX2 protease cleavage site were 5' TCG ACC GCG ACG GTG ACC GAC ACC TGG CGG AAG CGC CTA CAC TTA CTC CAA TGG GAA GAC TCG 3' (Seq ID No. 3) and 5' A ATT CGA GTC TTC CCA TTG GAG TAA GTG TAG GCG CTT CCG CCA GGT GTC GGT CAC CGT CGC GG 3' (Seq ID No. 4). The pair of complimentary oligonucleotides used to combine the two genes without the KEX2 protease cleavage site were as follows: 5' TCG ACC GCG ACG GTG ACC GAC ACC TGG CGG CTA CAC TTA CTC CAA TGG GAA GAC TCG 3' (Seq ID No. 5) and 5'A ATT CGA GTC TTC CCA TTG GAG TAA GTG TAG CCG CCA GGT GTC GGT CAC CGT CGC GG 3' (Seq ID No. 6). Both sets of oligonucleotide linkers were annealed and phosphorylated before ligation into the vector; pSL1180 (Pharmacia LKB Biotech, Piscataway, NJ. USA) digested with *Sal*I and *Eco*RI.

Several clones of the resulting plasmids, pSL1180:Sal-EcoR1 linker and pSL1180:Sal-EcoR1 kex2 linker, were sequenced and correct clones were identified. The correct clones of each were then digested with the restriction enzymes *Sal*I and *Eco*RI and the linkers of both types were isolated for ligations.

The vector, p:GAMpR was used to isolate a portion of the glucoamylase promoter, all of the glucoamylase terminator pBR322, and the *Aspergillus niger* var. *awamori pyrG* selectable marker by digesting it with the restriction enzymes *Mlu*I and *Xba*I, yielding a 9.6 kb and a 3.7 kb fragment. The 9.6 kb fragment was isolated for ligation. p: GAMpR was previously described in US Patent 5,364,770. Briefly, this plasmid contains the *Aspergillus niger* var. *awamori* UVK143f glucoamylase promoter, glucoamylase coding region fused to bovine chymosin coding region, glucoamylase terminator, and the *pyrG* selectable marker and bovine chymosin in a pBR322 backbone. The 9.6 kb *Mlu*I/*Xba*I fragment contained 1441 bases of the 5' portion of the glucoamylase promoter, the glucoamylase terminator, *pyrG*, and pBR322.

The vector pUC:AaGAM contained the *Aspergillus niger* var. *awamori* UVK143f glucoamylase promoter, glucoamylase structural gene, and glucoamylase terminator in a pUC vector. This was digested with restriction enzymes *Mlu*I and *Sal*I to yield a 6.3 and a 2.7 kb fragment. The 2.7 kb fragment, which contained 526 bases of the 3' end of the glucoamylase promoter and the whole coding region except for the final base pair, was isolated for ligation.

A four way ligation took place with all of the above isolated fragments: *Mlu*/*Xba* of p:GAMpR, the *Mlu*I/*Sal*I of pUC:AaGAM, the *Eco*RI/*Xba*I of pUC219:23ST RI/XbaI, and either the *Sal*-*Eco*RI linker or the *Sal*-*Eco*RI kex2 linker. The ligation produced several correct clones which were confirmed by restriction analysis. These plasmids were named pGAST (no KEX2 protease cleavage site) and pGASTK (with the KEX2 protease cleavage site).

The resulting plasmids were purified on a cesium chloride (CsCl) gradient in preparation for transformation into *Aspergillus niger* var. *awamori*.

Example 2

**Construction of pGADVST and pGADVSTK**

These plasmids were made to express the truncated form of alpha 2,3 sialyltransferase as a glucoamylase fusion in *Aspergillus niger* var. *awamori* similar to the constructs described in Example 1. However, in these constructs, the last 120 codons of the glucoamylase starch binding domain were omitted. Two approaches were taken to express sialyltransferase as a glucoamylase fusion. The first approach involved fusing the glucoamylase coding region from the *Nhe*I site which included all of the catalytic core and most of the glucoamylase linker region through amino acid #496 (alanine) to the sialyltransferase coding region from leucine #30. The second approach was nearly identical to the first approach with the addition of a KEX2 cleavage site in between the two coding regions. The following oligonucleotide pairs were used to fuse the two coding regions: 5' C TAGCAAAGCTACACTTACTCCAATGGGAAGACTCG 3' (Seq ID No. 7) and 5' AATTCGAGTCTTACCATTGGAGTAAGTGTAGCTTG 3' (Seq ID No. 8) for the construct without the KEX2 protease cleavage site and 5' CTAGCAAGCGCCTACACTTACTCCAATGGGAAGACTCG 3' (Seq ID No. 9) and 5' AATTCGAGTCTTACCATTGGAGTAAGTGTAGGCGCTTG 3' (Seq ID No. 10) for the construct which Included a KEX2 protease cleavage site in between the coding regions. The primer pairs were annealed by adding equal volumes of the primers into a tube and placing the tubes in a 100°C heat block for 2 minutes. The tubes were then slowly cooled to room temperature. The annealed primers were then phosphorylated as described by Maniatis et al. (in Molecular Cloning, A Laboratory Manual, 1980, Cold Spring Harbor Laboratory, Cold Spring Harbor, New York). The vector pSL1180 (Pharmacia LKB Biotech) was digested with *Nhe*I and *Eco*RI at 37°C for 1 hour and dephosphorylated with calf alkaline phosphatase (Boehringer Mannheim) for 1 hour, again at 37°C. Both sets of the annealed and phosphorylated linkers were ligated to the pSL1180 *Nhe*I/*Eco*RI digested vector in two separate ligations and transformed into *E. coli*. DNA was isolated from *E. coli* colonies obtained from these ligations and restriction endonuclease digestion identified potential correct clones of the *Nhe*I/*Eco*RI linkers were obtained and after DNA sequence analysis, correct linkers were identified. The plasmids containing the correct linkers were digested with *Nhe*I and *Eco*RI and subjected to electrophoresis on a 4% GTG NuSieve agarose gel (FMC Corporation, Rockland, Maine). After electroelution of the DNA bands containing the correct linkers from agarose, ethanol precipitation, and resuspension into TE buffer (10mMTris H-Cl, pH 7.6, 1 mM EDTA), the linkers were now ready for use.

An *Mlu*I/*Nhe*I digest was performed on pGAMpRpyr (described above) and the 2.3 kb fragment that included 526 bases of the *glaA* promoter and the glucoamylase coding region up to codon 496 was isolated on a 1% agarose gel and eluted. An *Eco*RI/*Nco*I digest of pUC219:23ST (described above) produced an 800 base pair sialyltransferase fragment that was missing the last 202 bases of the coding region. This fragment was isolated.

pSL1180 (Pharmacia LKB Biotech) was digested with *Mlu*I/*Nco*I, dephosphorylated, gel isolated and electroeluted. With each of the above isolated fragments and linkers, two four-way ligations were done. One set involved the linker without the KEX2 protease cleavage site and the other set involved the linker with this cleavage site. The resulting two plasmids, pSL:GADVST Part 1 and pSL:GADVSTK Part 1, were confirmed to be correct by restriction analysis.

Both plasmids were then digested with *Nhe*I and *Avr*II and an 80 base pair fragment was isolated from each plasmid which included the linker and 32 bases 3' from the EcoRI site of sialyltransferase. An *Nhe*I/*Avr*II digest was done on pSL:GASTpyrGV (refer to Figure 3C) to yield the large 11.3 kb vector fragment. pSL:GASTpyrGV was the result of subcloning a *Spe*I and *ClaI* fragment from pGAST into pSL1180. The *Spe*I/*Cla*I fragment contained approximately 800 bases of the 3' end of the promoter, the entire glucoamylase coding region, the truncated sialyltransferase gene and the glaA terminator. The resulting plasmid pSL:GAST was then digested with *Eco*RV, a unique site found 250 bases from the 5' end of the terminator. *A Sma*I/*Sca*I fragment containing the *A. niger pyrG* selectable marker was ligated into the pSL:GAST *EcoRV* digested vector resulting in pSL:GASTpyrGV.

Two separate ligations were performed with both 80 base pair linkers from pSL:GADVST Part 1 and pSL: GADVSTK Part 1 into the *Nhe*I/*Avr*II digested pSL:GASTpyrGV. The resulting plasmids, after confirmation by restriction analysis, were named pSL:GADVST Part 2 and pSL:GADVSTK Part 2, and each contained the glucoamylase promoter from the *Mlu*I site, the glucoamylase coding region without the starch binding domain, the linker with (pSL:GADVSTK) and without the KEX2 (pSL:GADVST) cleavage site, the truncated sialyltransferase gene and the *glaA* terminator. Both pSL:GADVST Part 2 and pSL:GADVSTK Part 2 were digested with *Mlu*I and a 3.3 kb fragment was isolated containing glucoamylase promoter and coding region without the starch binding domain, and sialyltransferase coding region minus the last 50 bases. Finally, pGASTK (described above) was digested with *Mlu*I and the 7.0 kb fragment which contained the rest of the *glaA* promoter of 526 bases, the *pyrG* selectable marker, and the last 50 bases of sialyltransferase was isolated. Both 3.3 kb *Mlu*I fragments of pSL:GADVST Part 2 and pSL:GADVSTK Part 2 were ligated into the 7.0 kb *Mlu*I fragment isolated from pGASTK. The final ligation yielded pGADVST and pGADVSTK. Both expression vectors were then grown for large scale isolation of the plasmid in order to obtain mass quantities of purified plasmid for fungal transformations.

<u>Example 3</u>

**Fungal Transformations**

CsCl gradient purified plasmid DNA was used to transform *Aspergillus niger* var. *awamori* dgr246 P2. dgr246 P2, described by Ward, M. et al. (1993) <u>Appl. Microbiol. Biotech.</u> **39**:738-743, is a strain derived from UVK143f that has been deleted of its major aspartic proteinase (aspergillopepsin) gene. Briefly, this strain had undergone several rounds of mutagenesis selection for improved expression of a heterologous gene product. The transformation protocol was a modification of the Campbell method (Campbell et al. (1989). <u>Curr. Genet.</u> **16**:53-56). All solutions and media were either autoclaved or filter sterilized through a 0.2 micron filter. Spores of dgr246 P2 were harvested off of complex media agar (CMA) plates. CMA contained 20 g/L dextrose, 20 g/L DifcoBrand malt extract, 1 g/L Bacto Peptone, 20 g/L Bacto agar, 20 ml/L of 100 mg/ml arginine, 20 ml/L of 100 mg/ml uridine, 1 ml/L of 10 mg/ml PABA, 10 ml/L of met/ bio solution, and 1 ml/L of 50 mg/ml streptomycin. Met/bio solution was composed of 50 g of L-methionine, 200 mg of D-biotin and distilled water up to 1 liter. An agar plug of approximately 1.5 cm square bearing sporulating mycelium was used to inoculate 100 mis of yeast extract broth with glucose (YEG) (5 g/L yeast extract, 20 g/L glucose, 20 ml/L 100 mg/ml arginine, 20 ml/L 100 ml/ml uridine, 1 ml/L 50 mg/ml streptomycin). The flask was incubated at 37°C on a shaker at 250-275 rpm, overnight. The mycelia were harvested through sterile Miracloth (Calbiochem, San Diego, CA, USA) in a funnel and washed with 200 mls of solution A (0.8 MgSO4 in 10 mM sodium phosphate, pH 5.8). The washed mycelia were placed in a sterile solution of 100 mg of Novozym234 (Novo Nordisk Industri A/S, Baagsvad, Denmark) in 20 mls of solution A. This was incubated at 28°C at 200 rpm for 1 hour in a sterile 250 ml bottle (Corning Inc., Corning, NY, USA). After incubation, the protoplasting solution was filtered through sterile Miracloth filters into a sterile 50 ml conical tube (Sarstedt, USA). The resulting liquid containing protoplasts was divided equally amongst four 50 ml conical tubes. Forty mls of solution B (1.2 M sorbitol, 50 mM $CaCl_2$, 10 mM Tris, pH 7.5) were added to each tube and centrifuged in a table top clinical centrifuge (Damon IEC HN SII centrifuge) at 3/4 speed for 10 minutes. The supernatant from each tube was discarded and 20 mls of fresh solution B was added to one tube, vortexed, then poured into the next tube until all the pellets were resuspended. The tube was then centrifuged at 3/4 speed for 10 minutes. The supernatant was discarded, 20 mls of fresh solution B was added, the tube was centrifuged for 10 minutes at 3/4 speed. The wash occurred one last time before resuspending the wash protoplasts into solution B, at 100 ul/0.5-1.0 X $10^7$ protoplasts. To each 100 ul of protoplasts in a sterile 15 ml conical (Sarstedt, USA), 10 ul of the transforming plasmid DNA was added. To this, 12.5 ul of solution C (50% PEG 4000, 50 mM $CaCl_2$, 10 mM Tris, pH 7.5) was added to the 15 ml tube and placed on ice for 20 minutes. One ml of solution C was added to the tube, removed from the ice to room temperature and mixed gently. Two mls of solution B were added immediately to dilute solution C. The transforming mix was added equally to 3 tubes of melted MMS overlay (6 g/L $NaNO_3$, 0.52 g/L KCl, 1.52 g/L $KH_2PO_4$, 218.5 g/L D-sorbitol, 1.0 ml/ L trace elements-LW, 10 g/L SeaPlaque agarose (FMC Bioproducts, Rockland, Maine, USA) 20 ml/L 50% glucose, 2.5 ml/L 20% $MgSO_4$*$7H_2O$, 10 ml/L met/bio solution, 2.0 ml/L 10 mg/ml PABA pH to 6.5 with NaOH) that were stored in a 37°C water bath. Trace elements LW consisted of 1 g/L $FeSO_4$*$7H_2O$, 8.8 g/L $ZnSO_4$*7 $H_2O$, 0.4 g/L $CuSO_4$*5

$H_2O$, 0.15 g/L $MnSO_4$*4 $H_2O$,0.1 g $Na_2B_4O_7$*10 $H_2O$, 50 mg/L $(NH_4)_6$ $Mo_7O_24$*4 $H_2O$, 250 mls $H_2O$, 200 ul/L concentrated HCl. The melted overlays with the transformation mix were immediately plated onto 3 MMS plates (same as MMS overlay recipe with the exception of 20 g/L of Bacto agar instead of 10 g/L of SeaPlaque) that had been supplemented with 200 ul/plate of 100 mglml of arginine added directly on top of the agar plate. After the agar solidified, the plates were incubated at 37°C until transformants grew.

The sporulating transformants were picked off with a sterile toothpick onto a plate of minimal media + glucose (MM). MM consisted of 6 g/L NaNO3, 0.52 g/L KCl, 1.52 g/L KH2PO4, 1 ml/L Trace elements LW, 20 g/L Bacto agar, pH to 6.5 with NaOH, 25 ml/L 40 % glucose, 2.5 ml/L 20% MgSo4*7H2O, 1 ml/L 50 mglml streptomycin plus 10 mg/ml carbenicillin, 20 ml/L 100 mg/ml arginine. Once the transformants grew on MM, they were transferred to a CMA plate, one transformant per plate of CMA.

Example 4

**Screening of Sialyltransferase Transformants**

A 1.5 cm square agar plug of each transformant was grown in 50 mls in a 250 ml shake flask of inoculum built-up medium, CSL + 5% xylose (100 g/L corn steep liquor (50% solids), National), 1 g/L $NaH_2PO_4$*$H_2O$, 0.5 g/L $MgSO_4$, 50 g/L xylose, 3 g/L mazu (Mazur Chemicals, USA), pH to 5.8 with NaOH, 1 ml/L 50 mg/ml streptomycin plus 10 mg/ml carbenicillin. Transformants and the parent control dgr246 P2 were incubated at 25°C, 160 rpm, for 2 days. Five mls of the 2 day old medium were inoculated into 50 mls of Clofine special (70 g/L sodium citrate, 15 g/L $(NH_4)_2$ $SO_4$, 1 g/L $NaH_2$ $PO_4$*$H_2O$, 1 g/L $MgSO_4$, 10 ml 10% Tween 80, 2 ml/L mazu, 60 g/L spray dried tofu TF30 soy milk powder (Armour Good Ingredients, Springfield, Kentucky, USA), 12% final concentration of maltose, 20 ml/L, 100 mg/ml arginine, 20 ml/L 100 mg/ml uridine, 10 ml/L met/bio solution, 1 mg/L streptomycin plus 10 mg/ml carbenicillin) in a 250 ml shake flask. The Clofine Special flasks were incubated at 25°C, 160 rpm for up to 7 days. Assays were performed on the supernatants of the growing cultures to determine the best producers of the enzyme of interest. The sialyltransferase assays were performed as described below.

Sialyltransferase Assay

The sialyltransferase assay was used to measure the enzymatic activity of alpha 2,3 sialyltransferase that catalyzed the following reaction:

**CMP-sialic acid + Lacto-N-Tetraose ——> CMP + sialyl lacto-N-tetraose**

The sialylated product was separated from unsialylated material on a column of ion exchange resin. Use of [$^{14}C$]-labeled substrate allowed measurement of the sialylated product by liquid scintillation counting. The resin used in the columns (Poly-prep chromatography columns 0.8 x 4 cm, Bio-Rad, Hercules, CA, USA) was AG1-X8, 200-400 mesh, chloride form (Bio-Rad). The resin was prepared by measuring out 100 g of the resin and then adding 400 mls of 1 M NaOH. The liquid was decanted and washed with 3 liters of distilled water. After the water was decanted, 400 mls of 1 M phosphoric acid was added, stirred and the resin was allowed to settle. The phosphoric acid was then decanted and the resin was washed with 3 liters of distilled water. After the water was decanted, 400 mls of 0.1 M sodium phosphate buffer, pH 6.8, was added and the resin and buffer slurry were further adjusted to pH 6.8. This was then washed a final time with 3 liters of distilled water and a final 50% slurry was made with MilliQ water. The slurry of prepared resin was swirled and 1 ml of the slurry was pipetted into a Bio-Rad column and allowed to settle.

The assay mix was made up of 0.5 mM cytidine 5' monophosphate sialic acid (CMP-SA) (Boehringer Mannheim, Indianapolis, IN, USA), 12.3 mM sodium cacodylate buffer, pH 6.0 (Sigma, St. Louis, MO, USA), 1.25% Triton CF-54 (Sigma), and 1.8 mg/ml bovine serum albumin (Sigma). The acceptor molecule, lacto-N-tetraose (LNT) (Sigma) was made to a 7 mM final concentration. The column buffer used to wash the resin was 5 mM sodium phosphate buffer, pH 6.8. The concentration of the assay mix was measured after dissolving the dry NEN-619 cytidine 5' monophosphate sialic acid (CMP-[$^{14}C$]-SA) (NEN Research Products, Dupont, USA) with 0.65 ml of the non-radioactive 5.76 mM CMP-SA. A 1:100 dilution of this was measured at OD270 against a $dH_2O$ blank. The CMP-SA concentration was calculated as follows:

[CMP-SA concentration (Molar) = (OD270)(1 mol/8600)(dilution factor)]

The assay mix was diluted so that when 25 ul of reaction mix were added to each sample, each sample received the equivalent of 3.4 ul of the above mixture. To the assay mixture, 960 ul 1 M NaCacodylate, 480 ul 20% Triton CF-54 and 290 ul 50 mg/ml BSA (filter sterilized) was added. Three hundred ul aliquots of this final mixture were stored at -20°C. The reaction mix was prepared by adding 250 ul of assay mix, 70 ul of 7 mM LNT, 12 ul of 1 M MnCl and 168 ul $dH_2O$ to an Eppendorf tube.
For each sample to be tested, 25 ul of reaction mix were added for each 14 ul of sample (supernatant from spent broth, standards and a buffer blank) to an Eppendorf tube. The tubes were spun down momentarily in a microcentrifuge and incubated in a 37°C water bath for 10 minutes. The standard alpha 2,3 sialyltransferase was provided by Cytel Corporation (San Diego, CA, USA) at 1 unit/ml. From this, dilutions using the sodium phosphate buffer were made for a range of 20-140 milli units, to generate a standard curve. Dilutions of the spent supernatants were also made in the sodium phosphate buffer. After incubation, 1 ml of column buffer was added to each tube and each sample was passed over an equilibrated column of resin. The unbound product was collected into a scintillation tube, washed with another 1 ml of column buffer, collected again and pooled. To each tube, 5 mls of aqueous scintillation fluid (ICN Ecolume, Costa Mesa, CA, USA) were added, shaken vigorously and counted in a liquid scintillation counter (TM Analytical) for 1 minute. The formulae required for the calculations of sialyltransferase activity units were as follows:

a) Total nmol CMP-SA in reaction = CMP-SA X 3.4 ul
where CMP-SA concentration = molar concentration as determined above;
b) cpm/nmol = total reaction cpm/nmol CMP-SA
where the total reaction cpm = total counts added to the reaction, corrected for blank and nmol CMP-SA = total nmol CMP-SA in the reaction as calculated in a); and finally
c) Activity (units/ml) = umol/min/ml =
[(sample cpm)/(cpm/nmol)](1/10min)(dilution factor)(1/0.014ml)(1.6)(1 umol/1000 nmol)
sample cpm = average cpm of sample corrected for average blank cpm
10 minutes = reaction incubation time
0.005 = volume of enzyme dilution tested
1.6 = unit definition was specified at saturating substrate concentration. Since the standard assay contained a subsaturating level of LNT, activity was multiplied by a factor of 1.6 to reflect maximal velocity by analysis of kinetics.

Example 5

**Screening of ST Transformants**

Following the transformation methods detailed in Example 3, twenty transformants with the pGAST expression vector, 22 with the pGASTK expression vector, 9 with the pGADVST expression vector, and 12 with the pGADVSTK expression vector were screened in shake flasks. Using the sialyltransferase activity assay of Example 4, the following transformant exhibited the best sialyltransferase levels secreted by transformants with each expression vector for pGAST transformant #20, 270 units/liter, for pGASTK transformant #4, 210 units/liter, for pGADVST#6, 120 units/liter and for pGADVSTK #1, 543 units/liter. Media optimization work as described in Example 6 was done with pGADVSTK#1 (DVK1).

Example 6

**Media and Process Development for the Production of Sialyltransferase in *Aspergillus***

**a.** Inoculum Medium

The medium used for the generation of cell mass was composed of: 100 g/L corn steep liquor; 0.5g/L magnesium sulfate; 1 g/L monobasic sodium phosphate monohydrate; 3 ml/L Mazu; 50 g/L xylose. The medium pH was adjusted to 5.8. The inoculum medium was inoculated with the desired culture and incubated at 25°C for 48 hr at 250 rpm. After the completion of the incubation time a given volume would be transferred to the production stage.

**b.** Production Medium

The initial medium used for the media development work was a liquid production medium, "Clofine Special." Clofine Special contained 60 g/L spray dried soy milk; 15 g/L ammonium sulfate; 70 g/L sodium citrate; 1 g/L magnesium sulfate; 1 ml/L Tween 80; 1 ml/L Mazu; 0.5 g/L potassium chloride; 1 g/L monobasic sodium phosphate monohydrate; 2 g/L arginine; 2 g/L uridine; and 120 g/L maltose. The pH of the medium was adjusted to 6.2. The production media

was inoculated with 5 mls of the desired culture, previously grown in the inoculum media. The production media was incubated the same as the inoculum media, except for a longer period of time.

X-Stat, a statistical design software, was used to conduct a number of experiments directed at the optimization of the production of glycosyltransferase, and particularly ST. After several experiments using X-Stat a new media evolved. The new medium contained: $KH_2PO_4$, 1 g/L; $MgSO_4$, 0.8 g/L; KCl, 1 g/L; arginine, 4 g/L; Tween 80, 1 ml/L; Mazu, 13 ml/L; spray dried soy milk, 60 g/L; maltose, 100 g/L; glucose, 10 g/L; ammonium sulfate, 13 g/L; sodium citrate, 60 g/L.

It was determined that the ST was aggregating to the spray dried soy milk in the media. Experiments were designed to look for an alternative complex nitrogen source for the medium. The new medium recipe described above was used to examine different nitrogen sources by substituting spray dried soy milk with wheat flour, Primetone CLT, Primetone SG, corn gluten and Sheftone. DVK1 was grown in media containing the various nitrogen sources and it was determined that Sheftone N provided a good source of complex nitrogen, and additionally added stability to the ST produced in the medium. Thus, this Sheftone N-containing medium had the advantage of improving the expression of the ST, as well as provided advantages during the recovery process of the protein (less degradation during recovery). Additional experiments followed this finding. Experiments were set up to study different concentrations of Sheftone N in the media. Results indicated that various concentrations of Sheftone N resulted in good expression, while concentrations from about 40 g/L up to about 100 g/L (preferably ≥80 g/L of Sheftone N) resulted in increased production of ST. The data are shown in Figure 4.

Additionally, during media optimization it was determined that the pH of the medium had a very important effect on the production and stability of the ST produced. Experiments studying pH were conducted. Results indicated that by increasing the pH of the medium over about pH 6 up to 8, increased levels of ST were produced. See Figure 5.

**c.** Buffer Concentration Studies

Based on the results shown in Figure 5, which indicated the pH of the medium had an effect on the production of ST, and knowing that the desired pH range for the medium was neutral, an experiment was conducted to study the buffer capacity of the medium. Sodium citrate which was present in the medium at an initial concentration of 60 g/L (see above) was increased up to at least 120 g/L. The results, as shown in Figure 6, indicated that stronger buffering capacity in the medium gave higher production of ST.

**d.** Process Development/14 L Fermentation Experiments

In order to scale up the results described above, Sheftone N-containing media (without the spray dried soy milk) at a concentration of 100 g/L Sheftone N and 60 g/L sodium citrate was used. The pH was adjusted to 6.5 and controlled at 5.8 with ammonia hydroxide. The fermenter produced 988 units/ml of ST. The yield from the fermentation came very close to the theoretical results based on the work presented here of 1057 units/ml. This represents a 93.5% duplication efficiency.

Example 7

**Construction of pGAKHi+**

**The Expression Vector Used to Express Fucosyltransferase**

An expression plasmid was constructed using methods known in the art, which allows DNA sequences encoding desired polypeptides to be fused to the C-terminus of the catalytic core and part of the linker region of *Aspergillus* glucoamylase.

The plasmid pGAKHi+ was constructed based on the bacterial plasmid pSL 1180 (Pharmacia LKB). This plasmid is similar to the pUC series of phagemids such as pUC118 but has an extended multiple cloning site (MCS) containing all possible six base pair palindromes including two recognition sites for *Cla*I, one of which is methylated and the other not methylated by *E. coli dam* methylase. An expression cassette was inserted into the MCS of pSL1180 so that the first 28 bp of the MCS from *Hin*dIII to *Spe*I was present at one end. Beginning at the *Spe*I recognition site, the expression cassette contains the following components: the promoter and 5' flanking region of the *glaA* gene of *A. niger* var. *awamori* strain UVK143f (US Patent 5,364,770) starting at a SpeI site approximately 1 kb upstream from the translation start codon; the coding region of the *glaA* gene from strain UVK 143f from the start codon to an *NheI* site, the six bp which encode amino acids 497 and 498 in the linker region of mature glucoamylase; a synthetic piece of double stranded DNA with *Nhe*I and *Bst*EII ends; approximately 250 bp of the terminator region of the *glaA* gene from *A. niger* strain #7 (US Patent 5,364,770) beginning at a *Bst*EII site 20 bp 5' of the translation stop codon and ending

at an *Eco*RV site; an approximately 2.4 kb fragment containing the *A. niger pyrG* gene (Wilson et al., 1988) obtained as a *Sma*I to *Sca*I fragment from pBH2 (Ward et al., 1989) (the *Sma*I site exists within the pUC18 MCS of pBH2 and the *Sca*I site exists 65 bp from the end of the *Bam*Hi-*Hin*dIII fragment of *A. niger* DNA present in pBH2); approximately 1.3 kb of 3' flanking DNA from the *glaA* from *A. niger*, beginning at the *Eco*RV site 250 bp 3' of the translation stop codon and ending at a *Cla*I site. After the *Cla*I site there exists 75 bp of the pSL 1180 MCS from *Cla*I (the site not methylated by *E. coli dam* methylase) to *Hin*dIII, followed by bp of pBR322 from *Hin*dIII to *Cla*I, followed by 198 bp of the pSL1180 MCS from *Cla*I (the site methylated by *E. coli dam* methylase) to *Eco*RI.

As shown in Figure 7 this expression vector (pGAKHi+) contains unique *Nhe*I and *Bst*EII sites which can be utilized to insert a DNA fragment encoding a desired polypeptide such that a fusion protein (comprising the glucoamylase catalytic core, part of the glucoamylase linker region and the desired polypeptide) would be expected to be produced after transformation into *Aspergillus* strains. The *pyrG* gene was included as a selectable marker for transformation into *Aspergillus* strains which are *pyrG* null mutants.

Example 8

**Construction of pGAKHi-FT**

Source of the FT cDNA

The human α1,3 fucosyltransferase V (FT) cDNA was obtained from Dr. John Lowe at the University of Michigan. The clone pcDNA1-FT contains the full-length FT sequence in pcDNA1 vector (Weston, B.W.; Nair, R.P.; Lowe, J.B. (1991) J. Biol. Chem. **267**:4152-60). The full-length done encompasses an amino-terminal cytoplasmic tail, a transmembrane signal anchor, a stem region, and a carboxy-terminal catalytic domain as typical of glycosyltransferases (Paulson, J.C.; Colley, K.J. (1989) J. Biol. Chem. **264**:17615-17618.)

Construction of pGAKHi-FT Plasmid

The construct was designed so that the *A. niger* glucoamylase gene would be fused in frame to the catalytic domain of the FT gene. The FT gene was amplified using native Pfu polymerase (Stratagene) and the polymerase chain reaction (PCR) using Buffer #1 and conditions supplied by the manufacturer. The primers generate an NheI restriction site at the 5' terminus and a BstEII site at the 3' terminus. The 5' primer was designed to hybridize to the stem portion of the FT gene, which fuses in-frame to the *A. niger* glucoamylase sequence of pGAKHi+ (Example 8), with a *kex2* proteolytic cleavage site between the glucoamylase and FT genes. The 3' primer was designed to amplify the 3' end of the FT coding region including the stop codon. The 5' and 3' primers were 5'-CTA-GCT-AGC-AAG-CGC-GGA-TCC-CCT-AGG-CCA-3' (Seq ID No. 11) and 5'-CCC-GGT-GAC-CTC-AGG-TGA-ACC-AAG-CC-3' (Seq ID No. 12), respectively. The PCR procedure amplified the stem region and catalytic domain of the FT gene, yielding a 1.2 kb fragment.

The amplified FT gene was sequentially digested with the enzymes Nhe I and BstEII (New England Biolabs), using conditions provided by the manufacturer, and fractionated on an agarose gel. The 1.2 kb fragment was isolated using a Prep-A-Gene kit (BioRad) and ligated under standard conditions into the unique NheI/BstEII sites of plasmid vector pGAKHi+. This fused the stem region of the FT gene in-frame with the glucoamylase sequence, generating pGAKHi-FT (Figure 8). Plasmids were transformed into *E. coli* strain DH5α, and ampicillin-resistant colonies were screened for the presence of the FT gene. The glucoamylase-FT fusion junction was sequenced using a primer that hybridizes to the glucoamylase sequence (5'-CCG-AGT-ATC-GTG-GCT-ACT-GGC-GGC-3' (Seq ID No. 13)). The plasmid of the correct construct was purified using Qiaex matrix (Qiagen).

Example 9

**Screening of FT Transformants**

Transformants were grown on agar plates of MM containing 5% xylose for 8 days at 37°C to generate spores. The spores were harvested in 0.1 % Tween/20% glycerol and stored at -70°C until used as stocks.

Spores (0.3 ml) from stocks were inoculated into 250 ml baffled flasks containing 50 mls of CSL + 5% xylose; cultures were grown at 37°C, 130 rpm for 2 days. This culture was used as the inoculum for the production medium.

Two production media, Clofine Special (Example 7) and Sheftone N (Example 7), were used for screening transformants. A 10% inoculum of CSL culture was inoculated into 50 mls of production medium in 250 ml baffled flasks. Cultures were grown at 32°C, 130 rpm for 4 days, at which time supernatants were assayed.

The protocol for the FT assay is provided below and is available from Cytel, San Diego. CA. The assay

measures the FT enzymatic activity and catalyzes the following reaction: GDP-fucose + N-acetyllactosamine ---> GDP + fucosyl N-acetyllactosamine. The fucosylated product was separated from GDP-fucose on an ion exchange column resin (AG1 X 8, chloride form). Use of $^{14}C$-labeted substrate (GDP-[$^{14}C$] fucose) allowed measurement of the fucosylated product by liquid scintillation counting.

| Fucosyltransferase Assay Protocol | |
|---|---|
| Cocktail Mix | Final Concentration |
| 70 ul [$^{14}C$]GDP-fucose (ca. 1.5 x $10^6$ cpm) Evaporate with $N_2$<br>5.5 ul cold GDP-fucose (6 mg/ml)<br>35 ul 1 M Na Cacodylate, pH 6.0<br>13 ul 1 M $MnCl_2$<br>130 ul 100 mM N-acetyllactosamine<br>400 ul $dH_2O$<br>584 ul total | 0.3 nmoles<br>4.0 nmoles<br>50 mM<br>20 mM<br>20 mM |
| Assay | |
| 45 ul cocktail mix<br>5 ul diluted enzyme<br>50 ul total per tube | |

Preheat tube with 45 ul reaction mix, 37°C for ca. 5 min
Add 5 ul diluted enzyme; time reactions
Incubate 10 min at 37°C
Terminate with 1 ml $dH_2O$
Run through AG1-X8 (chloride form) column (1.5 ml of 50:50 slurry)
Wash 1 time with 1 ml $dH_2O$
Count

- Run negative control
- Count 45 ul cocktail mix control for determining specific activity

Example 10

**Media Optimization for Increasing FT Expression**

Various parameters were tested for increasing FT expression in transformant KK17. Temperature, pH, incubation time, agitation speed, inoculum size, and soy protein source were among the variables tested. Several of these parameters affected the level of FT expression. For example, the optimum agitation speed for FT activity was about 180-220 rpm (Figure 9). In addition, pH affected expression, having an optimum value at pH 7.0 (Figure 10). Doubling the inoculum size from 10 to 20% increased activity at pH 7.0, 220 rpm from 580 U per liter to 690 U per liter.

Example 11

**Recombinant Baculovirus Expression System**

Construction of Recombinant Baculovirus

The recombinant baculovirus expressing ST was constructed as described in Ichikawa et al. (1992, J. Am. Chem. Soc. **114**:9283-9298). The gene was fused to the human insulin leader sequence, which allows the glycosyltransferase to be secreted. The baculovirus construction made for FT expression was similar, except the cDNA was cloned into the vector pVL1393, as illustrated in Fig. 11.

Stock cultures of *Spodoptera frugoperda* insect cells (Sf9II; Gibco/BRL) were grown at 27.5°C in 2-liter Fembach flasks containing 1 liter of Sf900 II serum-free media (Gibco/BRL). Circularized pVL1393-FT or pBlueBac-ST (2 mg) was co-transfected with linearized BaculoGold viral DNA (0.25 mg; PharMingen) into Sf9II cell monolayers using the transfection protocol of Invitrogen. The media containing recombinant virus particles was harvested after 5 days

incubation at 27°C. To isolate single viral clones, 2.5 x $10^3$ Sf9II cells were infected with serial 5-fold dilutions (12 each) of virus in 96-well limiting dilution plates (O'Reilly, D.R., Miller, L.K., Luckow, V.A., in Baculovirus Expression Vectors, A Laboratory Manual, W.H. Freeman and Co., 1992). Virus from wells exhibiting cell lysis at the end-point dilution were amplified by infecting 5 x $10^5$ cells with 0.2 pfu per cell in 24-well dishes. The amplified dones were then tested for activity: 5 x $10^5$ cells were infected with 3-5 pfu per cell and dishes were incubated for 48 hrs. All of the clones were positive for activity, and one done was used for the generation of the viral stock (passage 2), which was made in monolayer as follows: 3.0 x $10^7$ cells were absorbed to a T225 $cm^2$ tissue culture flask and infected with 0.2 pfu virus per cell for 1 hr at room temperature. Fresh Sf900II media (30 ml) was then added, and flasks were incubated for 48 hrs at 27.5°C. Virus was harvested by centrifugation (3700 rpm, 20 min). Supernatants were stored at 4C and used to make virus stocks for large scale protein production. Virus titer was determined by limiting dilution assay and was typically ca. 3 x $10^8$ pfu per ml.

Protein Production

Virus stocks (passage 3) were produced in shake flasks as follows: Sf9II cells were grown to 8 x $10^6$ cells per ml in 2 L Fembach flasks containing 0.5 L of Sf900II media. Virus from passage 2 was added at 0.2 pfu per cell; another 0.5 L of fresh Sf900II media was added, and flasks were incubated in shaking incubators (27.5°C, 130 rpm). Virus was harvested at 48 hrs by centrifugation (3700 rpm, 20 min), and supernatants were checked for contamination and stored at 4°C. Virus titers were determined by limiting dilution assay and were typically 2 x $10^9$ pfu per ml.

For protein production, Sf9 II cells (1 L) were grown at 27.5°C in 2 L cotton-stoppered Fembach flasks to ca. 8 x $10^6$ cells per ml. Cells were gently centrifuged (1000 rpm, 5 min) in sterile 1 liter bottles; supernatants were carefully dumped and virus was added to ca. 5-7 pfu per cell (ca. 25-50 ml). Fresh Sf900II medium was immediately added to restore the original volume, and cells were shaken at 27.5°C. Supernatants were harvested by centrifugation (3700 rpm; 15') at 48 hrs post-infection for ST or 72 hrs for FT.

**Example 12**

**Comparison of *A. niger* Expression System to Baculovirus Expression System**

Baculoviruses are insect-specific viruses that receive much interest for their use as recombinant protein expression systems. It is suggested to have a high rate and yield of expression, so it was desirable to compare it to the *A. niger* system described in this patent.

ST and FT activities in the two expression systems are shown in Table 2, which are the maximum activities over time.

Table 2

| **Maximum FT and ST Activities in a Recombinant Baculovirus Expression System as Compared to the A. niger Expression System** | | |
|---|---|---|
| System | ST (U/Liter) | FT (U/Liter) |
| Baculovirus | 140 | 50 |
| *A. niger* | 1700 | 600 |

The recombinant ST baculovirus produced 140 U/fiter, while the *A. niger* transformant produced 1700 U/liter. Likewise, the FT recombinant baculovirus expressed 50 U/liter, while FT *A. niger* transformant produced 600 U/liter. Both genes expressed 12-fold higher activities in *A. niger* than the corresponding baculovirus system.

The foregoing are presented by way of example only and should not be construed as a limitation to the scope of permissible claims.

Having described the preferred embodiments of the present invention, it would appear to those ordinarily skilled in the art that various modifications may be made to the disclosed embodiments, and that such modifications are intended to be within the scope of the present invention.

## Claims

1. A fusion DNA sequence encoding a fusion polypeptide comprising from the 5' end of said fusion DNA sequence:

   a) DNA encoding a signal peptide functional in *Aspergillus*;
   b) DNA encoding a secreted polypeptide or portion thereof normally secreted from *Aspergillus*;
   c) DNA encoding a cleavable linker polypeptide;
   d) DNA encoding a desired glycosyltransferase from which the transmembrane anchor coding sequence has been deleted

   wherein c) is optional.

2. A fusion DNA sequence of Claim 1 wherein the desired glycosyltransferase is selected from the group consisting of sialyltransferase, galactosyltransferase and fucosyltransferase.

3. A fusion DNA sequence of Claim 1 wherein the signal peptide is selected from the group consisting of signal peptides from glucoamylase, $\alpha$-amylase, and aspartyl protease from *Aspergillus* species and signal peptides from *Trichoderma* cellobiohydrolase I and II, and endoglucanase I and II.

4. A fusion DNA of Claim 3 wherein the signal peptide is the signal peptide from *A. niger* var. *awamori* glucoamylase.

5. A fusion DNA of Claim 1 wherein the secreted polypeptide or portion thereof is glucoamylase from *Aspergillus*.

6. A fusion DNA of Claim 5 wherein the secreted polypeptide is the full length mature glucoamylase from *A. niger* var. *awamori*.

7. A fusion DNA of Claim 5 wherein the secreted polypeptide is a portion comprising 50% of glucoamylase from *A. niger* var. *awamori*.

8. A fusion DNA of Claim 1 wherein the signal peptide comprises the signal peptide of *A. niger* var. *awamori* glucoamylase, the secreted polypeptide or portion thereof comprises glucoamylase from *A. niger* var. *awamori*, and the desired transferase is selected from the group consisting of sialyltransferase, galactosyltransferase and fucosyltransferase.

9. An expression vector for transforming an *Aspergillus* host cell comprising DNA sequences encoding regulatory sequences functionally recognized by the *Aspergillus* host, including promoter and transcription and translation initiation sequences operably linked to the 5' end of the fusion DNA sequence of Claim 1 and transcription stop sequences and polyadenylation sequences operably linked to the 3' end of said fusion DNA sequence.

10. An *Aspergillus* containing an expression vector of Claim 9.

11. A fusion polypeptide comprising from the 5' end:

    a) an amino acid sequence which is a signal peptide functional in *Aspergillus*;
    b) an amino acid sequence which is a secreted polypeptide or portion thereof normally secreted from *Aspergillus*;
    c) an amino acid sequence which is a cleavable linker;

d) an amino acid sequence which is a desired glycosyltransferase from which the transmembrane anchor region has been deleted

wherein c) is optional.

**12.** A fusion polypeptide of Claim 11 wherein the desired glycosyltransferase is selected from the group consisting of sialyltransferase, galactosyltransferase and fucosyltransferase.

**13.** A fusion polypeptide of Claim 11 wherein the signal peptide is selected from the group consisting of signal peptides from glucoamylase, $\alpha$-amylase, and aspartyl protease from *Aspergillus* species and signal peptides from *Trichoderma* cellobiohydrolase I and II, and endoglucanase I and II.

**14.** A fusion polypeptide of Claim 13 wherein the signal peptide is the signal peptide from *A. niger* var. *awamori* glucoamylase.

**15.** A fusion polypeptide of Claim 11 wherein the secreted polypeptide or portion thereof is glucoamylase from *Aspergillus*.

**16.** A fusion polypeptide of Claim 15 wherein the secreted polypeptide is the full length mature glucoamylase from *A. niger* var. *awamori*.

**17.** A fusion polypeptide of Claim 15 wherein the secreted polypeptide is a portion comprising 50% of glucoamylase from *A. niger* var. *awamori*.

**18.** A fusion polypeptide of Claim 11 wherein the signal peptide comprises the signal peptide of *A. niger* var. *awamori* glucoamylase, the secreted polypeptide or portion thereof comprises glucoamylase from *A. niger* var. *awamori*, and the desired glycosyltransferase is selected from the group consisting of sialyltransferase, galactosyltransferase and fucosyltransferase.

**Patentansprüche**

**1.** Fusions-DNA-Sequenz, kodierend für ein Fusionspolypeptid, umfassend vom 5'-Ende der Fusions-DNA-Sequenz:

a) DNA, kodierend ein Signalpeptid, das in Aspergillus funktionell ist;
b) DNA, kodierend ein sezerniertes Polypeptid oder einen Teil davon, normalerweise sezerniert von Aspergillus;
c) DNA, kodierend eine spaltbares Linkerpolypeptid;
d) DNA, kodierend eine gewünschte Glycosyltransferase, von der die den Transmembrananker kodierende Sequenz deletiert wurde,

wobei c) optional ist.

**2.** Fusions-DNA-Sequenz gemäß Anspruch 1, wobei die gewünschte Glycosyltransferase ausgewählt ist aus der Gruppe bestehend aus Sialyltransferase, Galactosyltransferase und Fucosyltransferase.

**3.** Fusions-DNA-Sequenz gemäß Anspruch 1, wobei das Signalpeptid ausgewählt ist aus der Gruppe bestehend aus den Signalpeptiden von Glucoamylase, $\alpha$-Amylase und Aspartylprotease von Aspergillus-Arten und Signalpeptiden von Trichoderma Cellobiohydrolase I und II und Endoglucanase I und II.

**4.** Fusions-DNA gemäß Anspruch 3, wobei das Signalpeptid das Signalpeptid von A. niger var. awamori Glucoamylase ist.

**5.** Fusions-DNA gemäß Anspruch 1, wobei das sezernierte Polypeptid oder das Teil davon Glucoamylase von Aspergillus ist.

**6.** Fusions-DNA gemäß Anspruch 5, wobei das sezernierte Polypeptid die reife volllängen-Glucoamylase von A. niger var. awamori ist.

7. Fusions-DNA gemäß Anspruch 5, wobei das sezertierte Polypeptid ein Teil ist, umfassend 50 % Glucoamylase von A. niger var. awamori.

8. Fusions-DNA gemäß Anspruch 1, wobei das Signalpeptid das Signalpeptid von A. niger var. awamori Glucoamalyse umfaßt, das sezernierte Polypeptid oder Teil davon umfaßt Glucoamylase von A. niger var. awamori und die gewünschte Transferase ist ausgewählt aus der Gruppe bestehend aus Sialyltransferase, Galactosyltransferase und Fucosyltransferase.

9. Expressionsvektor für die Transformation einer Aspergillus-Wirtszelle, umfassend DNA-Sequenzen, kodierend regulatorische Sequenzen, funktionell erkannt von dem Aspergillus-Wirt, einschließlich Promotor und Transkriptions- und Translations-Initiationssequenzen, operabel verbunden mit dem 5'-Ende der Fusions-DNA-Sequenz gemäß Anspruch 1, und Transkriptions-Stopsequenzen und Polyadenylierungssequenzen, operabel verbunden mit dem 3'-Ende der Fusions-DNA-Sequenz.

10. Aspergillus, enthaltend einen Expressionsvektor gemäß Anspruch 9.

11. Fusionspolypeptid, umfassend vom 5'-Ende her:

a) eine Aminosäuresequenz, die ein Signalpeptid, funktionell in Aspergillus, ist;
b) eine Aminosäuresequenz, die ein sezerniertes Polypeptid oder ein Teil davon, normalerweise sezerniert von Aspergillus, ist;
c) eine Aminosäuresequenz, die ein spaltbarer Linker ist;
d) eine Aminosäuresequenz, die eine gewünschte Glucosyltransferase ist, von der der Transmembran-Ankerbereich deletiert wurde,

wobei c) optional ist.

12. Fusionspolypeptid gemäß Anspruch 11, wobei die gewünschte Glucosyltransferase ausgewählt ist aus der Gruppe bestehend aus Sialyltransferase, Galatosyltransferase und Fucosyltransferase.

13. Fusionspolypeptid gemäß Anspruch 11, wobei das Signalpeptid ausgewählt ist aus der Gruppe bestehend aus den Signalpeptiden von Glucoamylase, α-Amylase und Aspartylprotease von Aspergillus-Arten und Signalpeptiden von Trichoderma Cellobiohydrolase I und II und Endoglucanase I und II.

14. Fusionspolypeptid gemäß Anspruch 13, wobei das Signalpeptid das Signalpeptid von A. niger var. awamori Glucoamylase ist.

15. Fusionspolypeptid gemäß Anspruch 11, wobei das sezernierte Polypeptid oder der Teil davon Glucoamylase von Aspergillus ist.

16. Fusionspolypeptid gemäß Anspruch 15, wobei das sezernierte Polypeptid die reife Volllängen-Glucoamylse von A. niger var. awamori ist.

17. Fusionspolypeptid gemäß Anspruch 15, wobei das sezernierte Polypeptid ein Teil ist, umfassend 50 % von Glucoamylase von A. niger var. awamori.

18. Fusionspolypeptid gemäß Anspruch 11, wobei das Signalpeptid das Signalpeptid von A. niger var. awamori Glucoamylase umfaßt, das sezernierte Polypeptid oder Teil davon umfaßt Glucoamylase von A. niger var. awamori, und die gewünschte Glucosyltransferase ist ausgewählt aus der Gruppe bestehend aus Sialyltransferase, Galactosyltransferase und Fucosyltransferase.

**Revendications**

1. Séquence d'ADN de fusion codant pour un polypeptide de fusion comprenant à partir de l'extrémité 5' de ladite séquence d'ADN de fusion :

a) un ADN codant pour un peptide signal fonctionnel dans *Aspergillus* ;

b) un ADN codant pour un polypeptide sécrété ou une partie de celui-ci sécrétée normalement par *Aspergillus* ;
c) un ADN codant pour un polypeptide lieur qui peut être coupé ;
d) un ADN codant pour une glycosyltransférase désirée de laquelle la séquence codante d'ancrage transmembranaire a été supprimée

dans laquelle c) est facultatif.

**2.** Séquence d'ADN de fusion de la revendication 1, dans laquelle la glycosyltransférase désirée est sélectionnée parmi le groupe consistant en sialyltransférase, galactosyltransférase et fucosyltransférase.

**3.** Séquence d'ADN de fusion de la revendication 1, dans laquelle le peptide signal est sélectionné parmi le groupe consistant en peptides signal de glucoamylase, _-amylase, et aspartylprotéase de l'espèce *Aspergillus* et en peptides signal de cellobiohydrolase I et II, et d'endoglucanase I et II *Trichoderma*.

**4.** ADN de fusion de la revendication 3, dans lequel le peptide signal est le peptide signal de la glucoamylase *A. niger* var. *awamori*.

**5.** ADN de fusion de la revendication 1, dans lequel le polypeptide sécrété ou une partie de celui-ci est la glucoamylase d'*Aspergillus*.

**6.** ADN de fusion de la revendication 5, dans lequel le polypeptide sécrété est la glucoamylase mature complète de *A. niger* var. *awamori*.

**7.** ADN de fusion de la revendication 5, dans lequel le polypeptide sécrété est une partie comprenant 50 % de glucoamylase de *A. niger* var. *awamori*.

**8.** ADN de fusion de la revendication 1, dans lequel le peptide signal comprend le peptide signal de la glucoamylase *A. niger* var. *awamori*, le polypeptide sécrété ou une partie de celui-ci comprend la glucoamylase de *A. niger* var. *awamori*, et la transférase désirée est sélectionnée parmi le groupe consistant en sialyltransférase, galactosyltransférase et fucosyltransférase.

**9.** vecteur d'expression pour transformer une cellule hôte *Aspergillus* comprenant des séquences d'ADN codant pour des séquences de régulation identifiées de manière fonctionnelle par l'hôte *Aspergillus*, incluant des séquences de début de traduction et de transcription et de type promoteur liées de façon opérationnelle à l'extrémité 5' de la séquence d'ADN de fusion de la revendication 1 et des séquences d'arrêt de transcription et des séquences de polyadénylation liées de façon opérationnelle à l'extrémité 3' de ladite séquence d'ADN de fusion.

**10.** Aspergillus contenant un vecteur d'expression de la revendication 9.

**11.** Polypeptide de fusion comprenant à partir de l'extrémité 5' :

a) une séquence d'acides aminés qui est un peptide signal fonctionnel dans *Aspergillus* ;
b)une séquence d'acides aminés qui est un polypeptide sécrété ou une partie de celui-ci sécrétée normalement par *Aspergillus* ;
c) une séquence d'acides aminés qui est un lieur qui peut être coupé ;
d)une séquence d'acides aminés qui est une glycosyltransférase désirée de laquelle la région d'ancrage transmembranaire a été supprimée dans laquelle c) est facultatif.

**12.** Polypeptide de fusion de la revendication 11, dans lequel la glycosyltransférase désirée est sélectionnée parmi le groupe consistant en sialyltransférase, galactosyltransférase et fucosyltransférase.

**13.** Polypeptide de fusion de la revendication 11, dans lequel le peptide signal est sélectionné parmi le groupe consistant en peptides signal de glucoamylase, -amylase, et aspartylprotéase de l'espèce *Aspergillus* et en peptides signal de cellobiohydrolase I et II, et d'endoglucanase I et II *Trichoderma*.

**14.** Polypeptide de fusion de la revendication 13, dans lequel le peptide signal est le peptide signal de la glucoamylase *A. niger* var. *awamori*.

**15.** Polypeptide de fusion de la revendication 11, dans lequel le polypeptide sécrété ou une partie de celui-ci est la glucoamylase d'*Aspergillus*.

**16.** Polypeptide de fusion de la revendication 15, dans lequel le polypeptide sécrété est la glucoamylase mature complète de *A. niger* var. *awamori*.

**17.** Polypeptide de fusion de la revendication 15, dans lequel le polypeptide sécrété est une partie comprenant 50 % de glucoamylase de *A. niger* var. *awamori*.

**18.** Polypeptide de fusion de la revendication 11, dans lequel le peptide signal comprend le peptide signal de la glucoamylase *A. niger* var. *awamori*, le polypeptide sécrété ou une partie de celui-ci comprend la glucoamylase de *A. niger* var. *awamori*, et la glycosyltransférase désirée est sélectionnée parmi le groupe consistant en sialyltransférase, galactosyltransférase et fucosyltransférase.

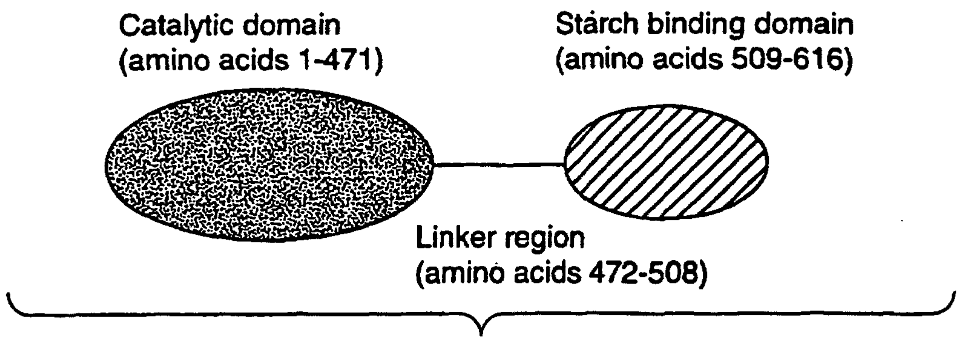
Catalytic domain
(amino acids 1-471)
Starch binding domain
(amino acids 509-616)
Linker region
(amino acids 472-508)

FIG._1

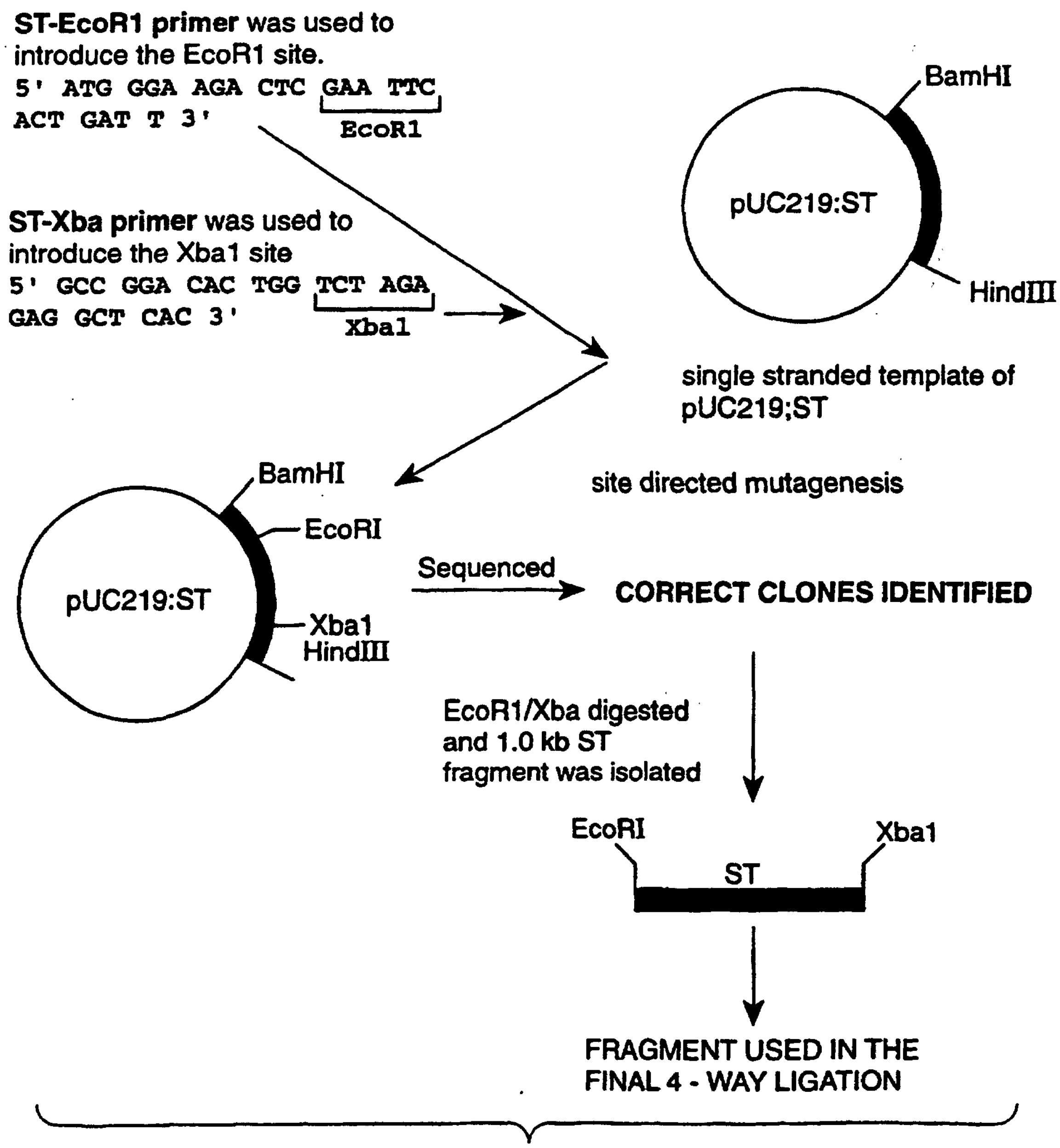
ST-EcoR1 primer was used to introduce the EcoR1 site.
5' ATG GGA AGA CTC GAA TTC ACT GAT T 3'
EcoR1
ST-Xba primer was used to introduce the Xba1 site
5' GCC GGA CAC TGG TCT AGA GAG GCT CAC 3'
Xba1
BamHI
pUC219:ST
HindIII
single stranded template of pUC219;ST
site directed mutagenesis
BamHI
EcoRI
pUC219:ST
Xba1
HindIII
Sequenced
CORRECT CLONES IDENTIFIED
EcoR1/Xba digested and 1.0 kb ST fragment was isolated
EcoRI
Xba1
ST
FRAGMENT USED IN THE FINAL 4 - WAY LIGATION

FIG._2A

**GAM-ST link Sal1-EcoR1 no kex**

TCG ACC GCG ACG GTG ACC GAC ACC TGG CGG CTA CAC TTA CTC CAA TGG GAA GAC TCG
AAT CGA GTC TTC CCA TTG GAG TAA GTG TAG CCG CCA GGT GTC GGT CAC CGT CGC GG

annealed and phosphorylated → Subcloned into pSL1180: NheI/EcoR1 → CORRECT CLONES VERIFIED BY SEQUENCING

annealed and phosphorylated → Subcloned into pSL1180: Nhe1/EcoR1 → CORRECT CLONES VERIFIED BY SEQUENCING

**GAM-ST link Sal1-EcoR1 with kex**

TCG ACC GCG ACG GTG ACC GAC ACC TGG CGG AAG CGC CTA CAC TTA CTC CAA TGG GAA GAC TCG
AATT CGA GTC TTC CCA TTG GAG TAA GTG TAG GCG CTT CCG CCA GGT GTC GGT CAC CGT CGC GG

***FIG._2B***

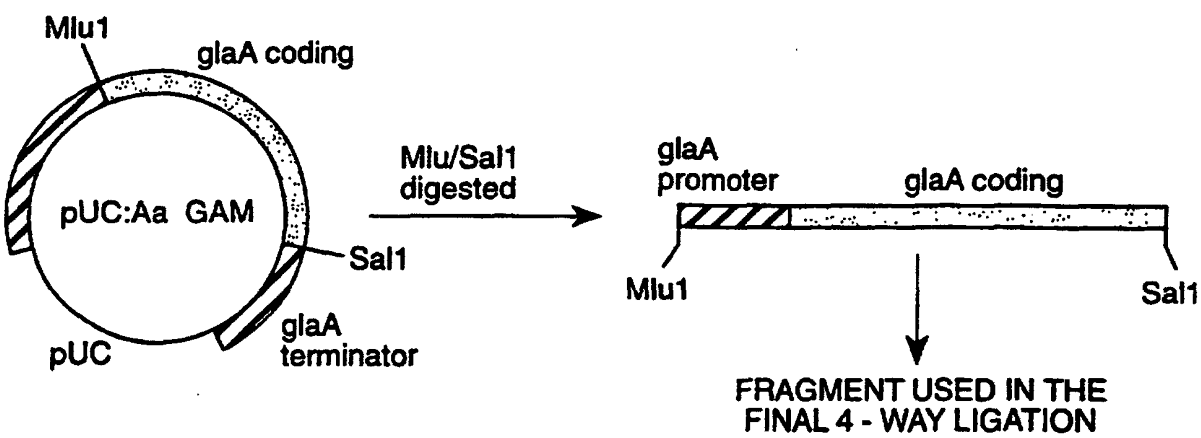


***FIG._2C***

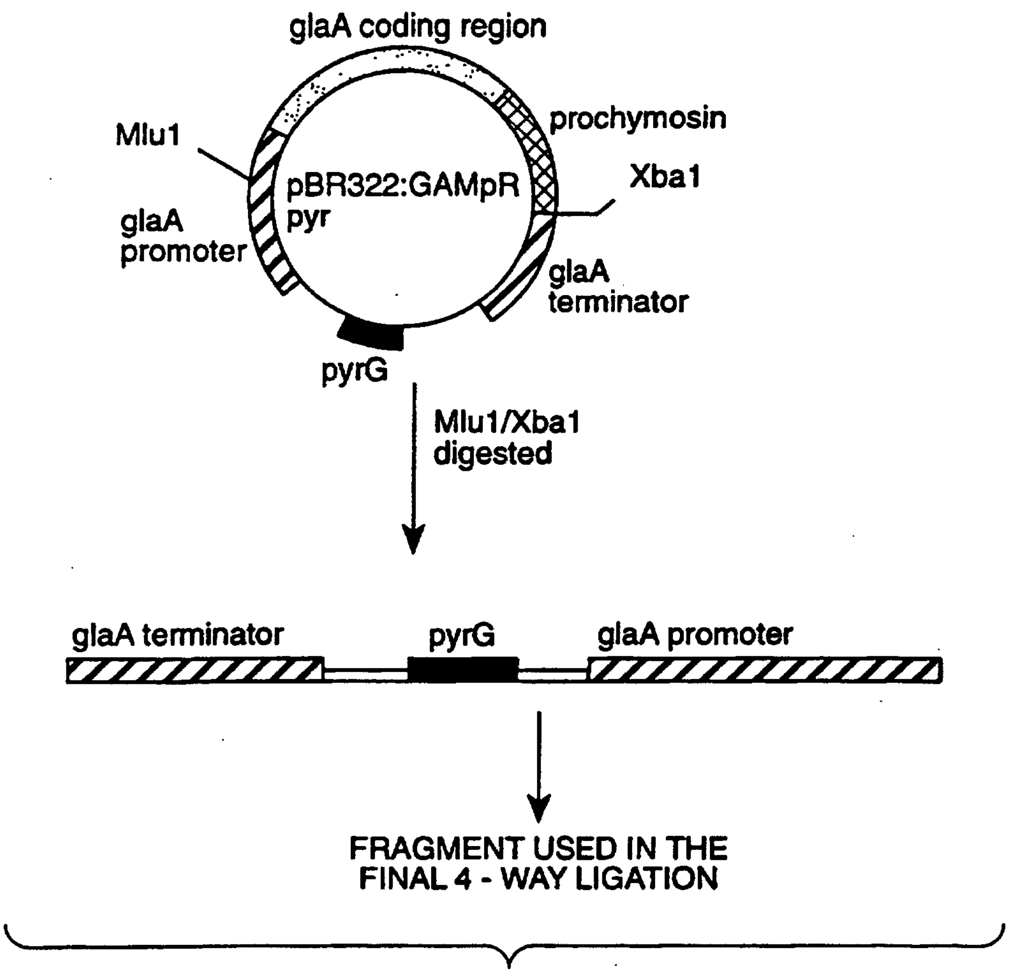
glaA coding region
prochymosin
Mlu1
pBR322:GAMpR
pyr
Xba1
glaA
promoter
glaA
terminator
pyrG
Mlu1/Xba1
digested
glaA terminator
pyrG
glaA promoter
FRAGMENT USED IN THE
FINAL 4 - WAY LIGATION

*FIG._2D*

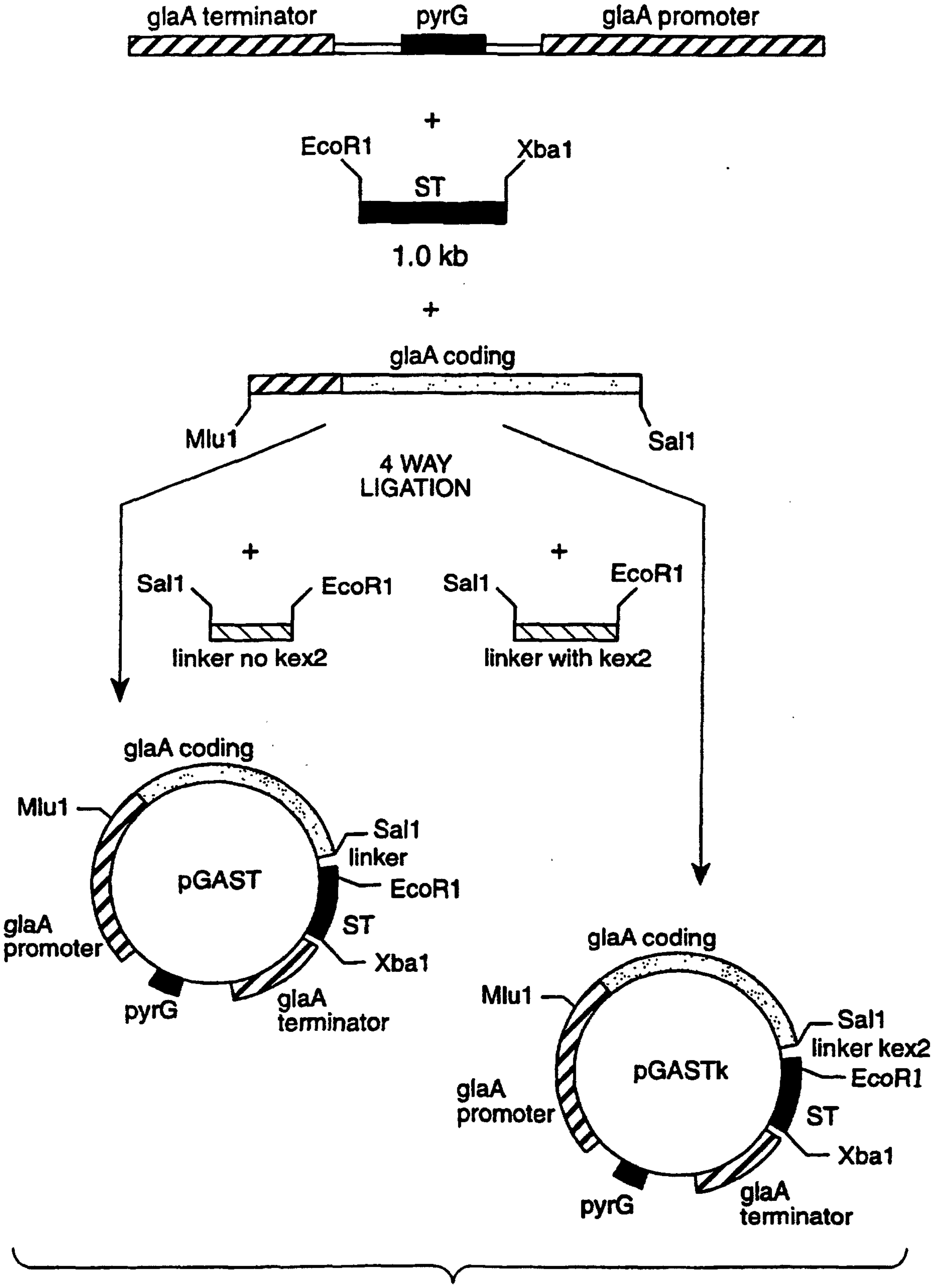
glaA terminator
pyrG
glaA promoter
+
EcoR1
Xba1
ST
1.0 kb
+
glaA coding
Mlu1
Sal1
4 WAY
LIGATION
+
Sal1
EcoR1
linker no kex2
+
Sal1
EcoR1
linker with kex2
glaA coding
Mlu1
Sal1
linker
pGAST
EcoR1
ST
glaA
promoter
Xba1
pyrG
glaA
terminator
glaA coding
Mlu1
Sal1
linker kex2
pGASTk
EcoR1
glaA
promoter
ST
Xba1
pyrG
glaA
terminator

*FIG._2E*

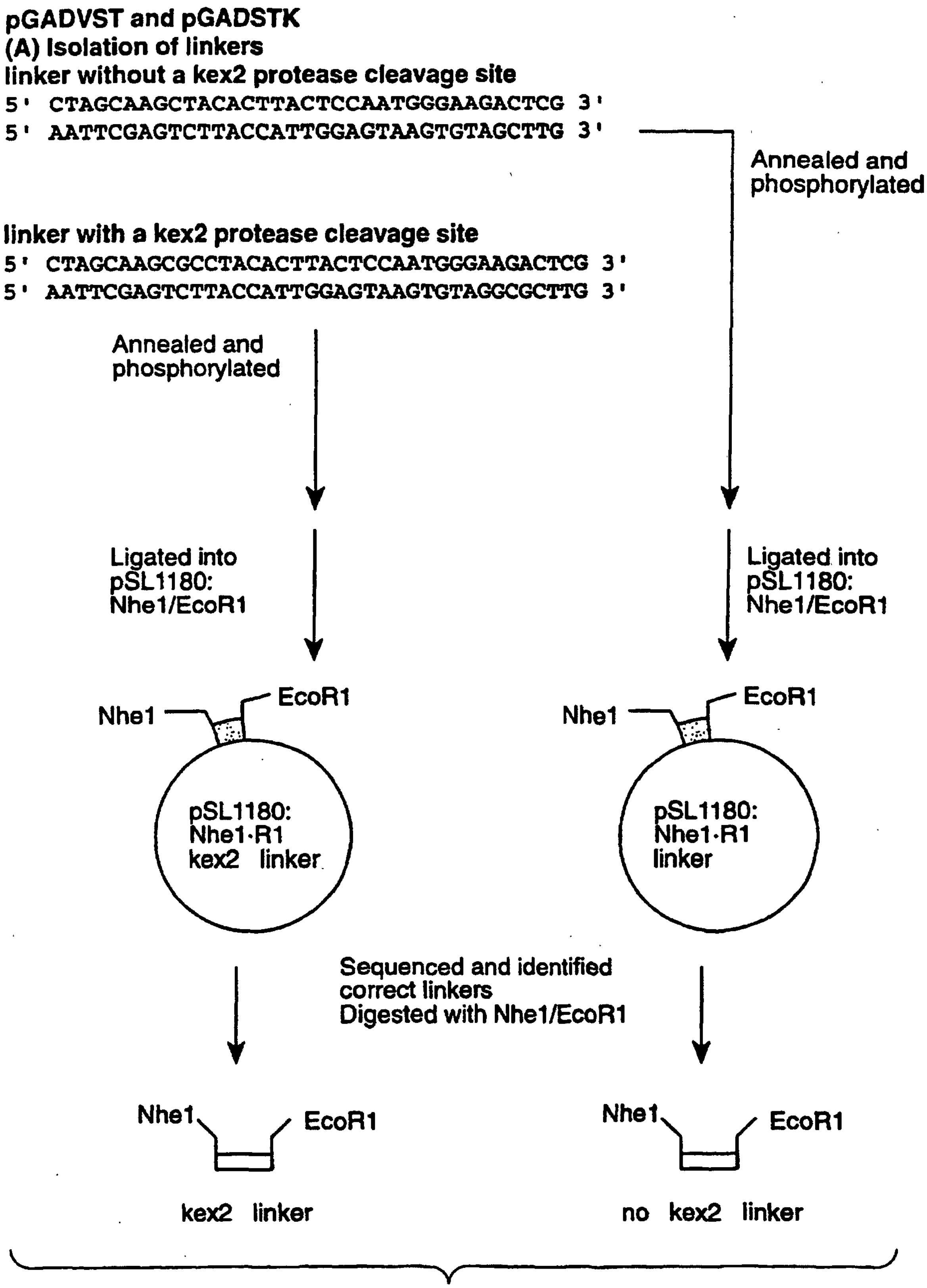
pGADVST and pGADSTK
(A) Isolation of linkers
linker without a kex2 protease cleavage site
5' CTAGCAAGCTACACTTACTCCAATGGGAAGACTCG 3'
5' AATTCGAGTCTTACCATTGGAGTAAGTGTAGCTTG 3'
Annealed and phosphorylated
linker with a kex2 protease cleavage site
5' CTAGCAAGCGCCTACACTTACTCCAATGGGAAGACTCG 3'
5' AATTCGAGTCTTACCATTGGAGTAAGTGTAGGCGCTTG 3'
Annealed and phosphorylated
Ligated into pSL1180: Nhe1/EcoR1
Ligated into pSL1180: Nhe1/EcoR1
Nhe1
EcoR1
pSL1180: Nhe1·R1 kex2 linker
Nhe1
EcoR1
pSL1180: Nhe1·R1 linker
Sequenced and identified correct linkers
Digested with Nhe1/EcoR1
Nhe1
EcoR1
kex2 linker
Nhe1
EcoR1
no kex2 linker

*FIG._3A*

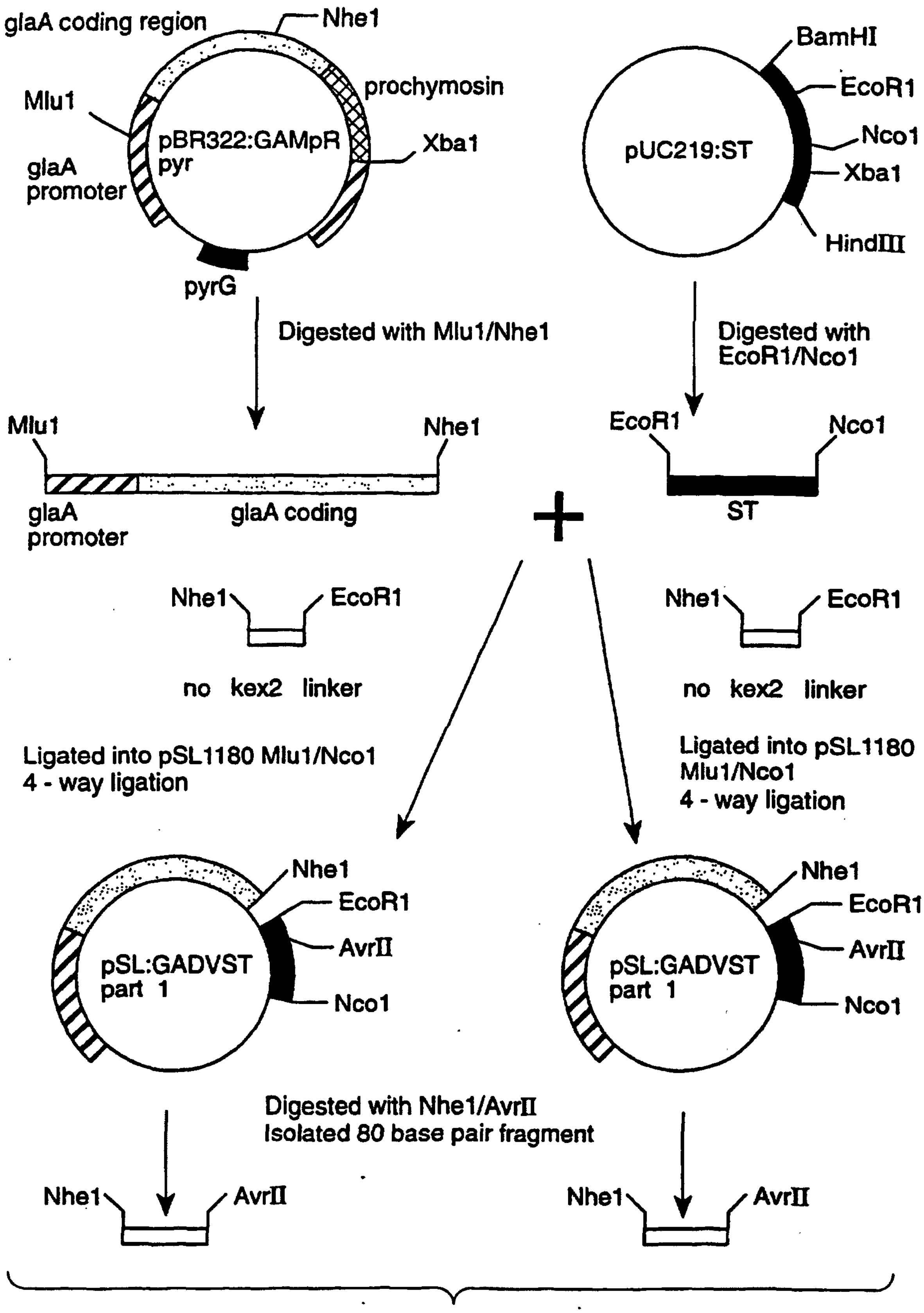
glaA coding region
Nhe1
Mlu1
prochymosin
pBR322:GAMpR
pyr
Xba1
glaA
promoter
pyrG
BamHI
EcoR1
Nco1
pUC219:ST
Xba1
HindIII
Digested with Mlu1/Nhe1
Digested with EcoR1/Nco1
Mlu1
Nhe1
EcoR1
Nco1
glaA
promoter
glaA coding
ST
Nhe1
EcoR1
no kex2 linker
Nhe1
EcoR1
no kex2 linker
Ligated into pSL1180 Mlu1/Nco1
4 - way ligation
Ligated into pSL1180
Mlu1/Nco1
4 - way ligation
Nhe1
EcoR1
AvrII
pSL:GADVST
part 1
Nco1
Nhe1
EcoR1
AvrII
pSL:GADVST
part 1
Nco1
Digested with Nhe1/AvrII
Isolated 80 base pair fragment
Nhe1
AvrII
Nhe1
AvrII

FIG._3B

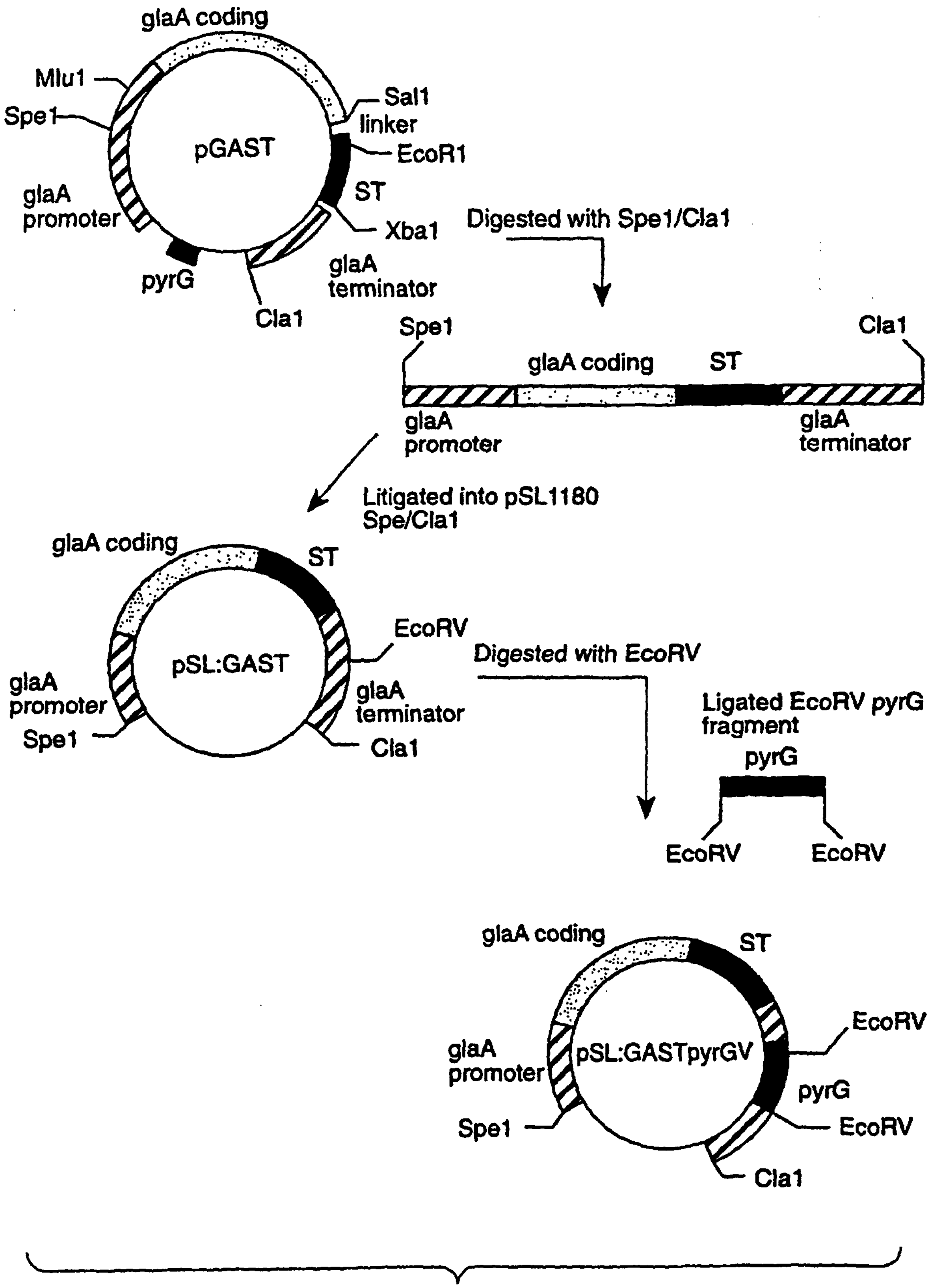
glaA coding
Mlu1
Spe1
Sal1
linker
pGAST
EcoR1
glaA
promoter
ST
Xba1
Digested with Spe1/Cla1
pyrG
glaA
terminator
Cla1
Spe1
Cla1
glaA coding
ST
glaA
promoter
glaA
terminator
Litigated into pSL1180
Spe/Cla1
glaA coding
ST
EcoRV
pSL:GAST
Digested with EcoRV
glaA
promoter
glaA
terminator
Ligated EcoRV pyrG
fragment
Spe1
Cla1
pyrG
EcoRV
EcoRV
glaA coding
ST
EcoRV
glaA
promoter
pSL:GASTpyrGV
pyrG
Spe1
EcoRV
Cla1

FIG._3C

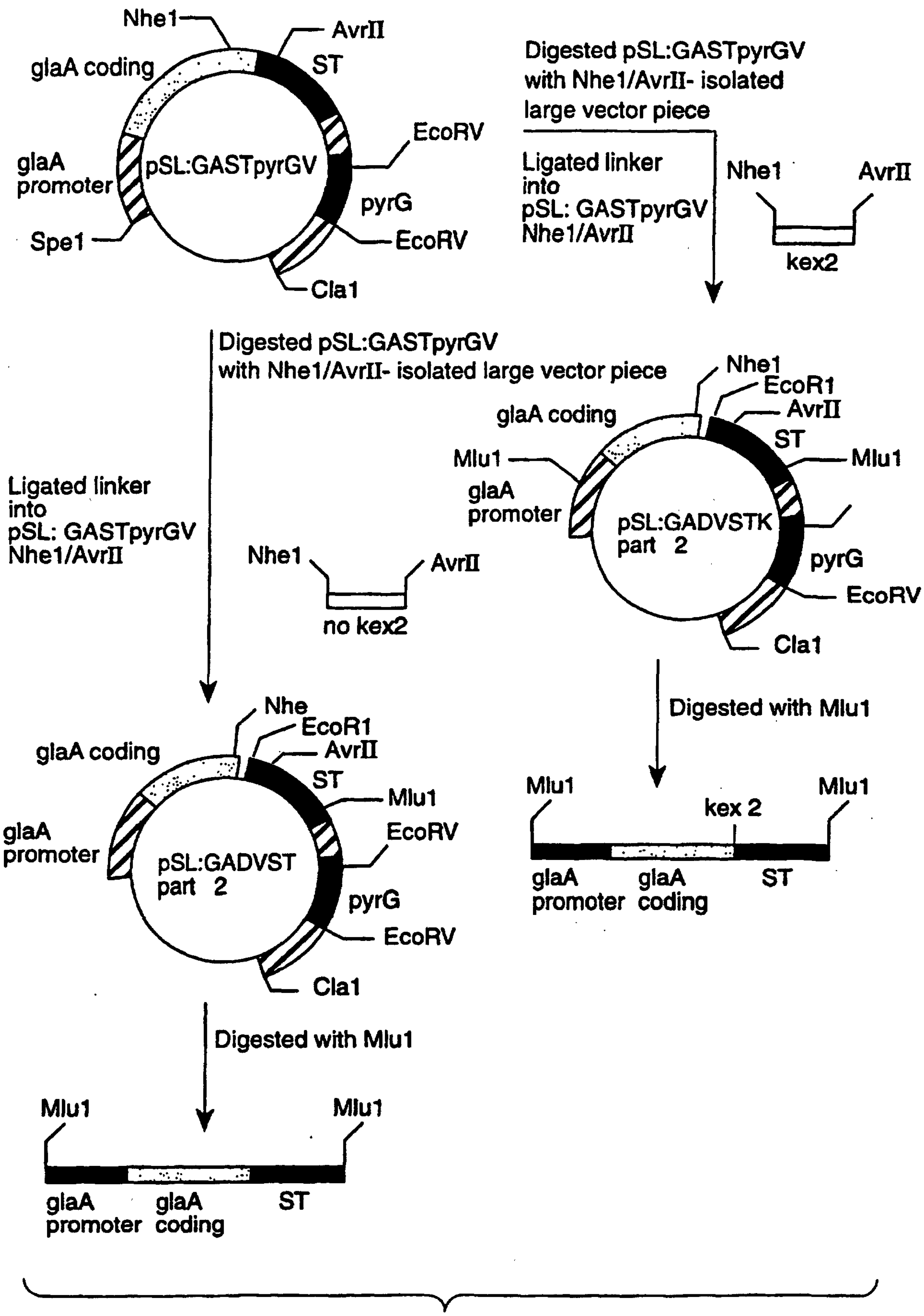
Nhe1
AvrII
glaA coding
ST
pSL:GASTpyrGV
EcoRV
glaA promoter
pyrG
Spe1
EcoRV
Cla1
Digested pSL:GASTpyrGV with Nhe1/AvrII- isolated large vector piece
Ligated linker into pSL: GASTpyrGV Nhe1/AvrII
Nhe1
AvrII
kex2
Digested pSL:GASTpyrGV with Nhe1/AvrII- isolated large vector piece
Nhe1
EcoR1
AvrII
glaA coding
ST
Mlu1
Mlu1
glaA promoter
pSL:GADVSTK part 2
pyrG
EcoRV
Cla1
Ligated linker into pSL: GASTpyrGV Nhe1/AvrII
Nhe1
AvrII
no kex2
Digested with Mlu1
Mlu1
Mlu1
kex 2
glaA promoter
glaA coding
ST
Nhe
EcoR1
AvrII
glaA coding
ST
Mlu1
EcoRV
glaA promoter
pSL:GADVST part 2
pyrG
EcoRV
Cla1
Digested with Mlu1
Mlu1
Mlu1
glaA promoter
glaA coding
ST

FIG_3D

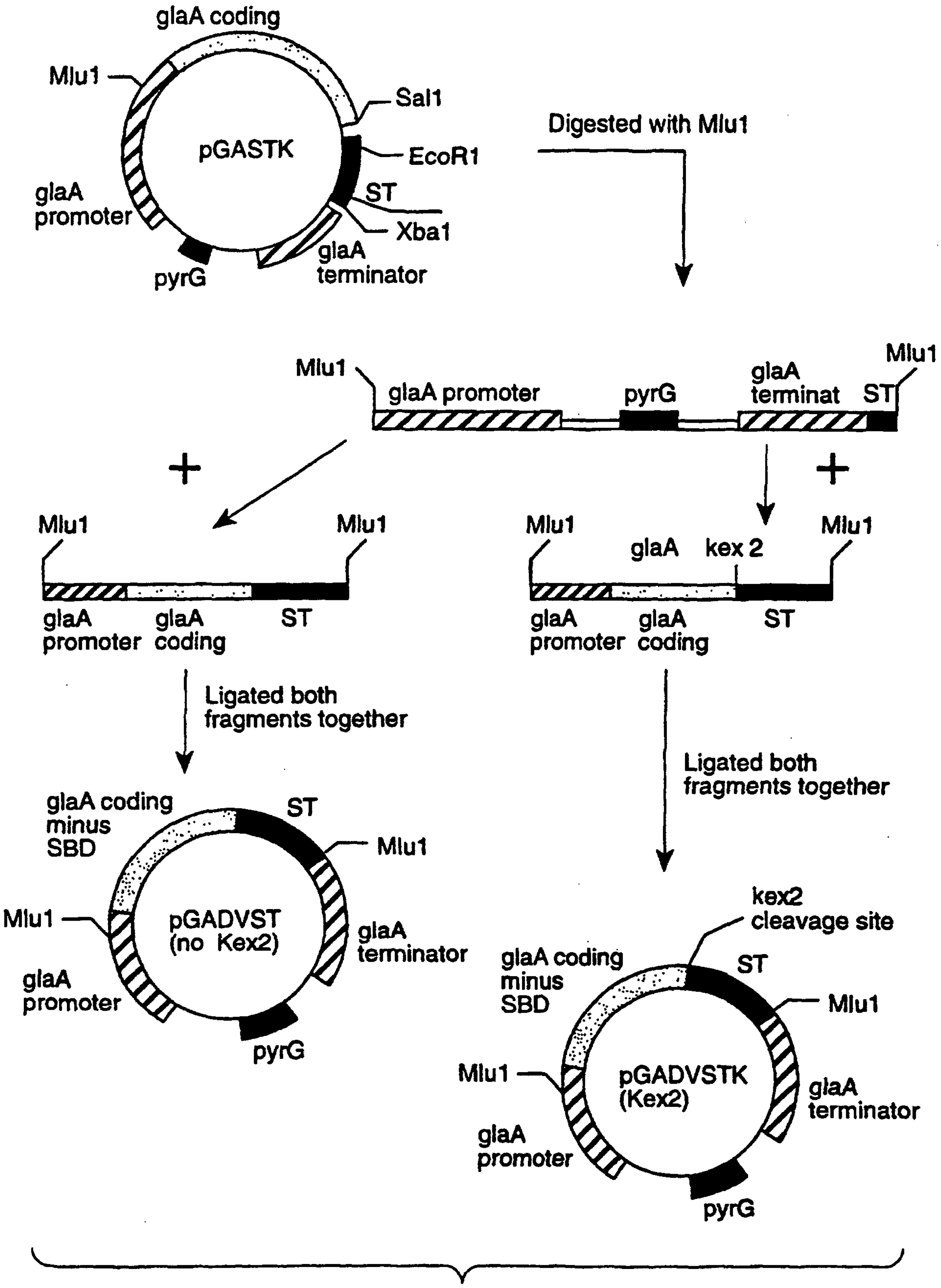
glaA coding
Mlu1
Sal1
pGASTK
EcoR1
glaA promoter
ST
Xba1
pyrG
glaA terminator
Digested with Mlu1
Mlu1
glaA promoter
pyrG
glaA terminat
ST
Mlu1
+
+
Mlu1
Mlu1
glaA promoter
glaA coding
ST
Mlu1
glaA
kex 2
Mlu1
glaA promoter
glaA coding
ST
Ligated both fragments together
Ligated both fragments together
glaA coding minus SBD
ST
Mlu1
Mlu1
pGADVST (no Kex2)
glaA terminator
glaA promoter
pyrG
kex2 cleavage site
glaA coding minus SBD
ST
Mlu1
Mlu1
pGADVSTK (Kex2)
glaA terminator
glaA promoter
pyrG

*FIG._3E*

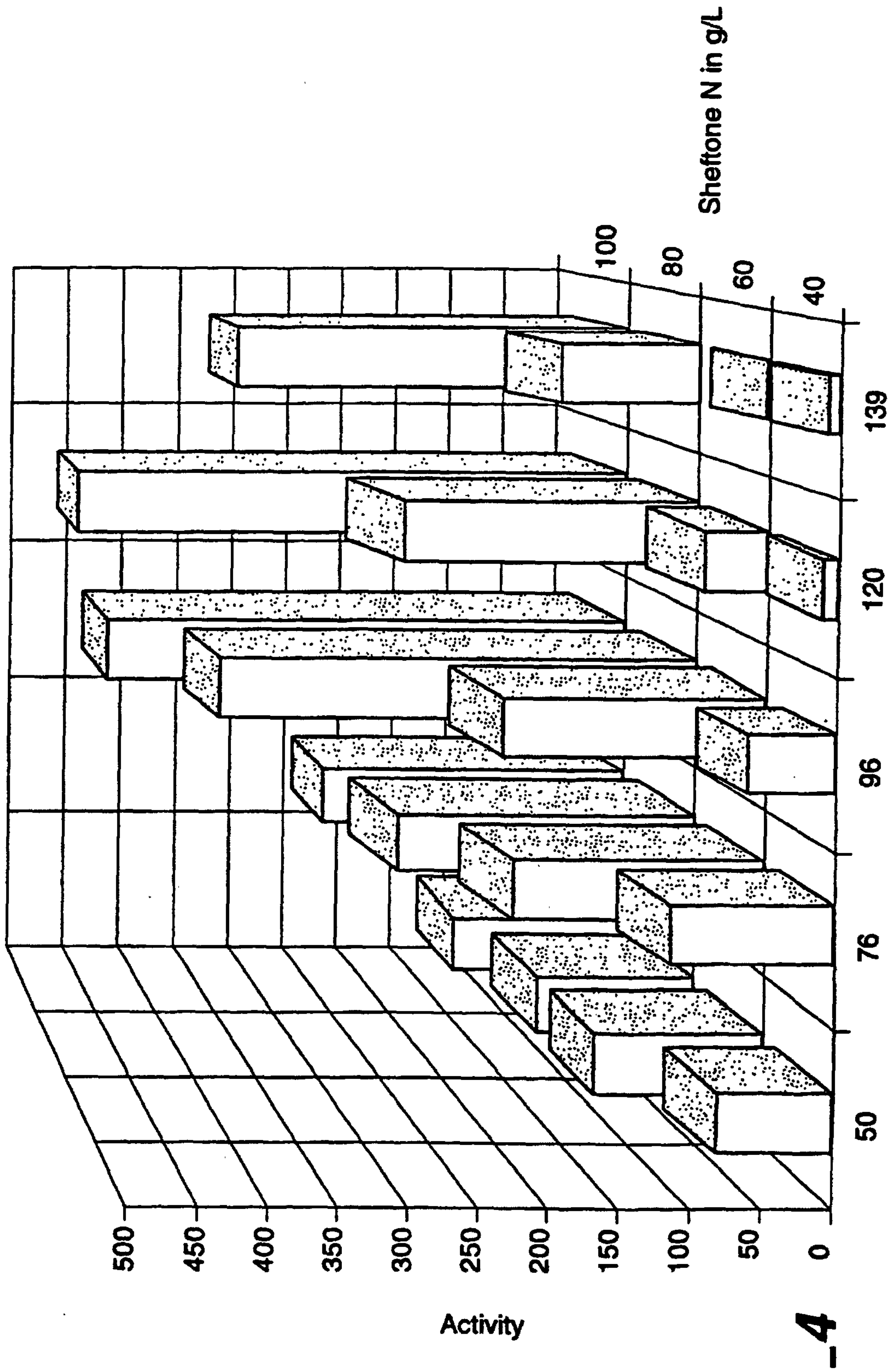
Activity
500
450
400
350
300
250
200
150
100
50
0
Time (hrs.)
50
76
96
120
139
Sheftone N in g/L
100
80
60
40

FIG._4

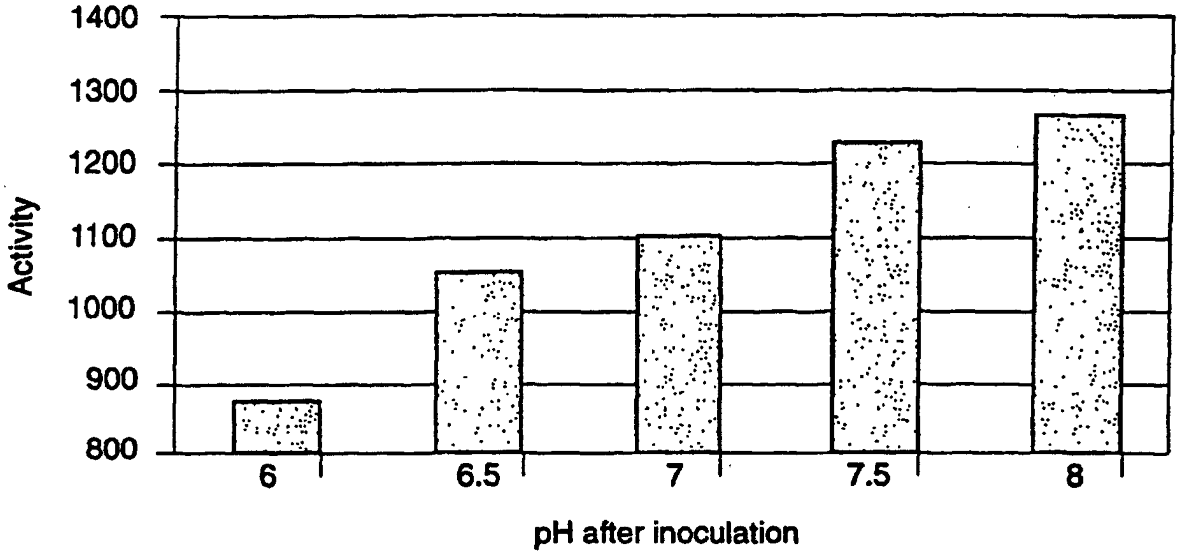
1400
1300
1200
1100
1000
900
800
Activity
6
6.5
7
7.5
8
pH after inoculation

FIG._5

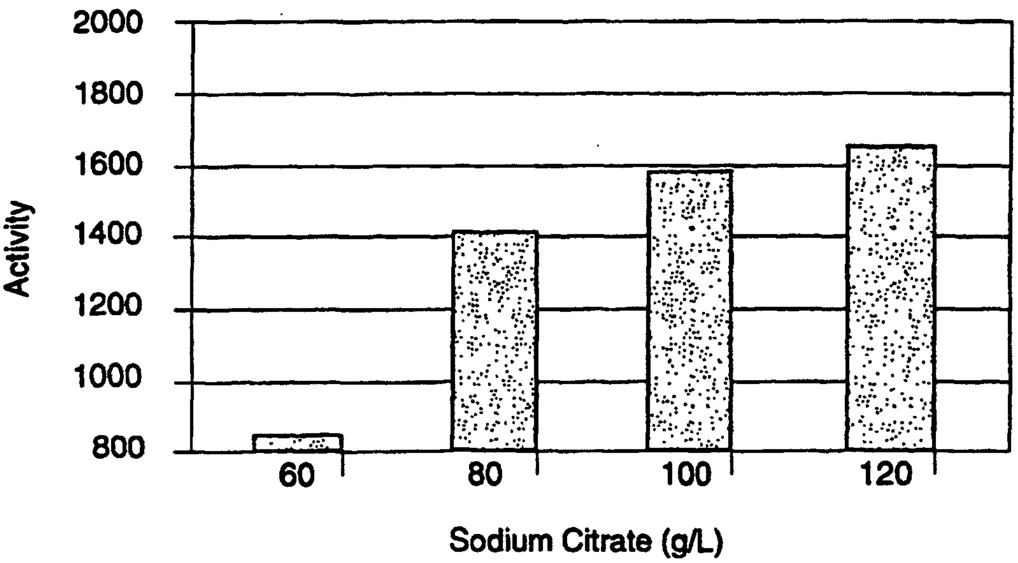
2000
1800
1600
1400
1200
1000
800
Activity
60
80
100
120
Sodium Citrate (g/L)

FIG._6

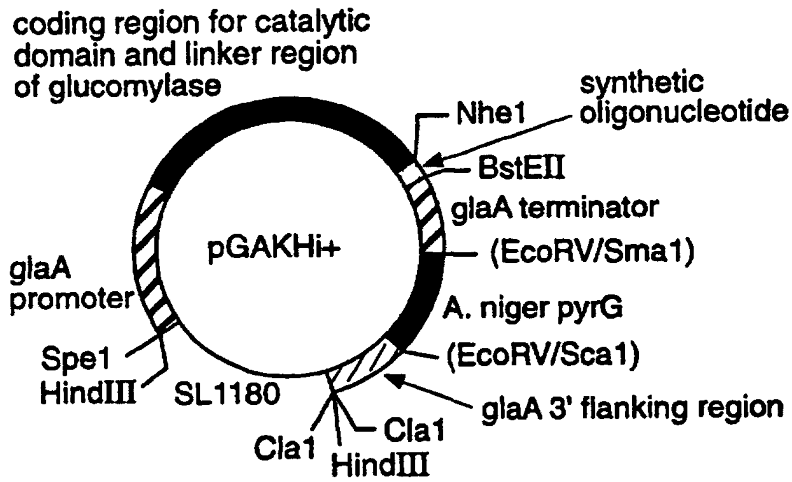
coding region for catalytic
domain and linker region
of glucomylase
Nhe1
synthetic
oligonucleotide
BstEII
glaA terminator
(EcoRV/Sma1)
pGAKHi+
glaA
promoter
A. niger pyrG
(EcoRV/Sca1)
Spe1
HindIII
SL1180
glaA 3' flanking region
Cla1
Cla1
HindIII

FIG._7

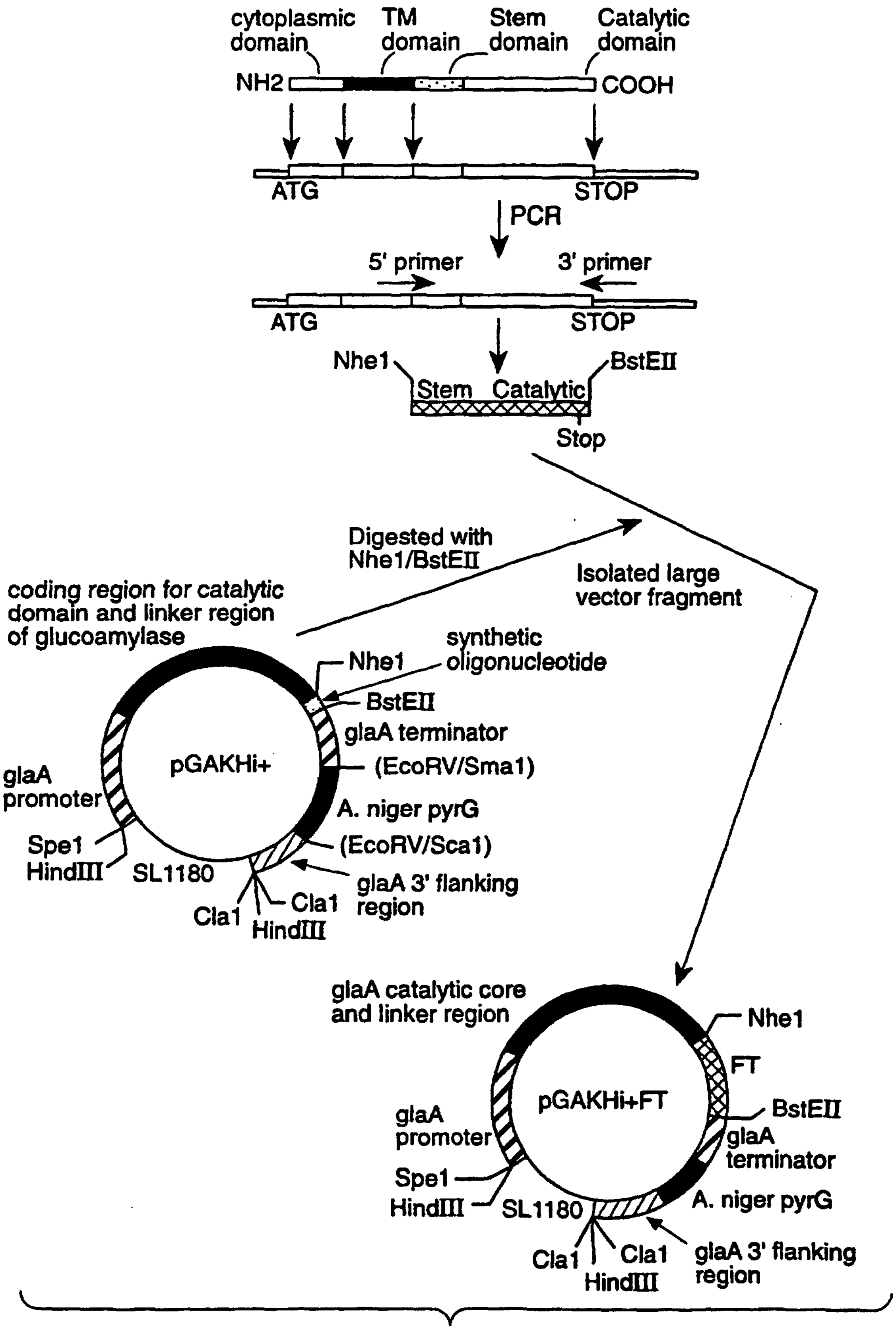
cytoplasmic domain
TM domain
Stem domain
Catalytic domain
NH2
COOH
ATG
STOP
PCR
5' primer
3' primer
ATG
STOP
Nhe1
BstEII
Stem Catalytic
Stop
Digested with Nhe1/BstEII
Isolated large vector fragment
coding region for catalytic domain and linker region of glucoamylase
Nhe1
synthetic oligonucleotide
BstEII
glaA terminator
(EcoRV/Sma1)
pGAKHi+
glaA promoter
A. niger pyrG
(EcoRV/Sca1)
Spe1
HindIII
SL1180
glaA 3' flanking region
Cla1
Cla1
HindIII
glaA catalytic core and linker region
Nhe1
FT
pGAKHi+FT
BstEII
glaA promoter
glaA terminator
Spe1
HindIII
SL1180
A. niger pyrG
Cla1
Cla1
HindIII
glaA 3' flanking region

*FIG._8*

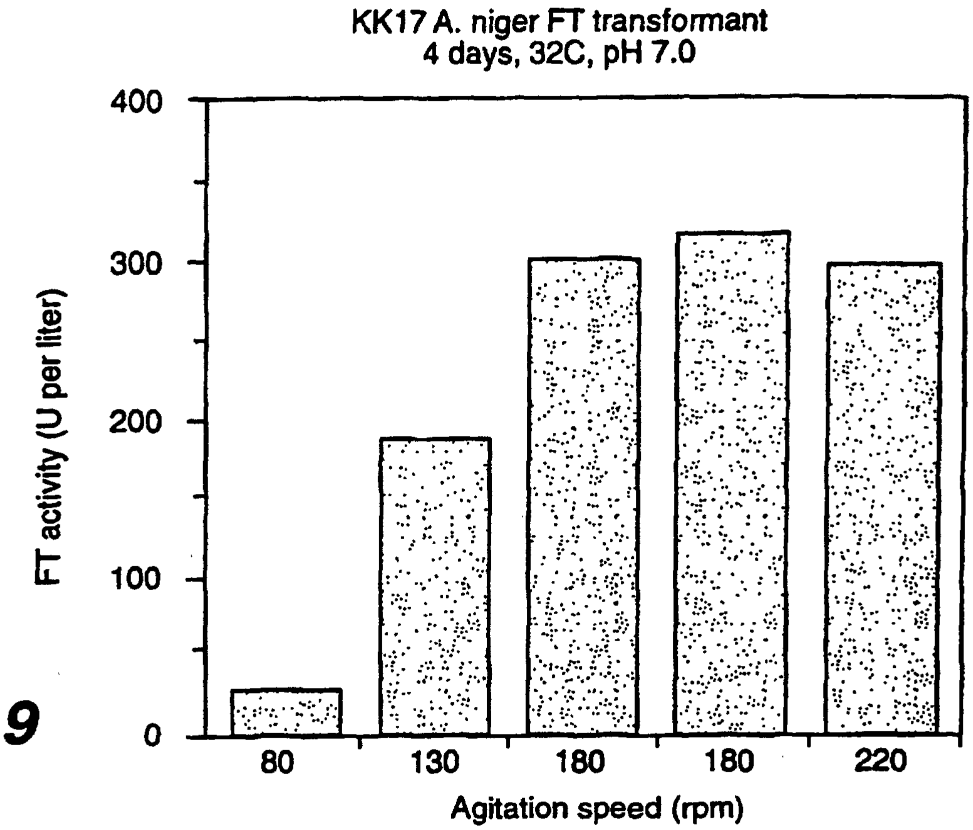
KK17 A. niger FT transformant
4 days, 32C, pH 7.0
FT activity (U per liter)
400
300
200
100
0
80
130
180
180
220
Agitation speed (rpm)

FIG._9

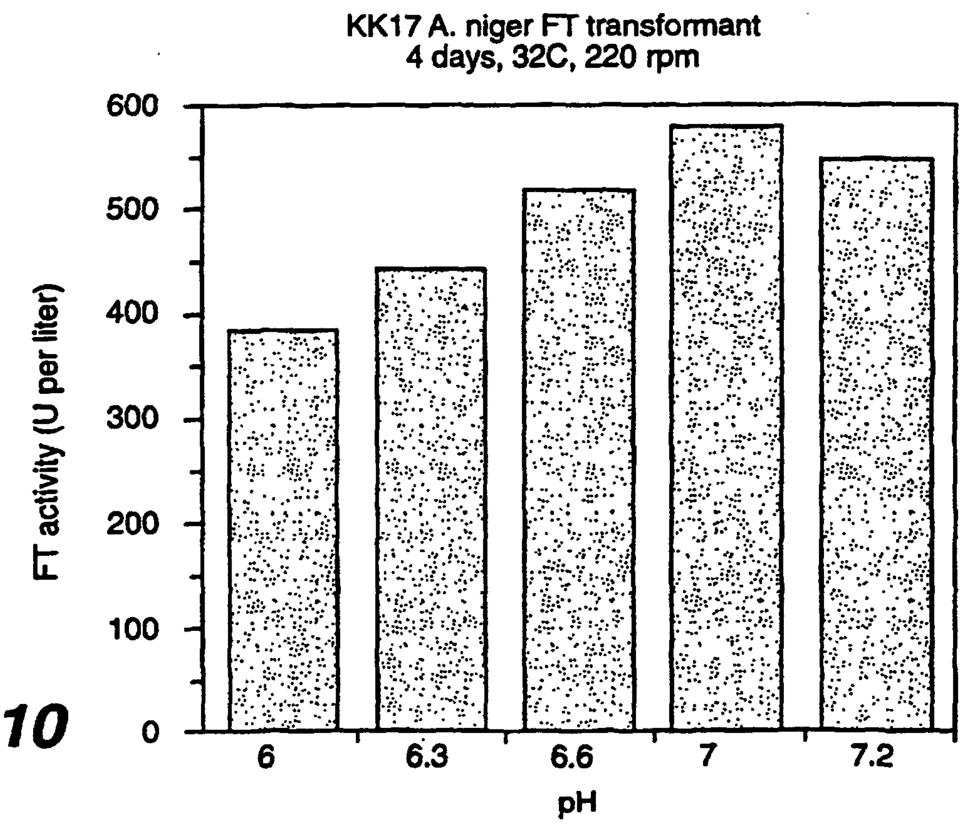
KK17 A. niger FT transformant
4 days, 32C, 220 rpm
FT activity (U per liter)
600
500
400
300
200
100
0
6
6.3
6.6
7
7.2
pH

FIG._10

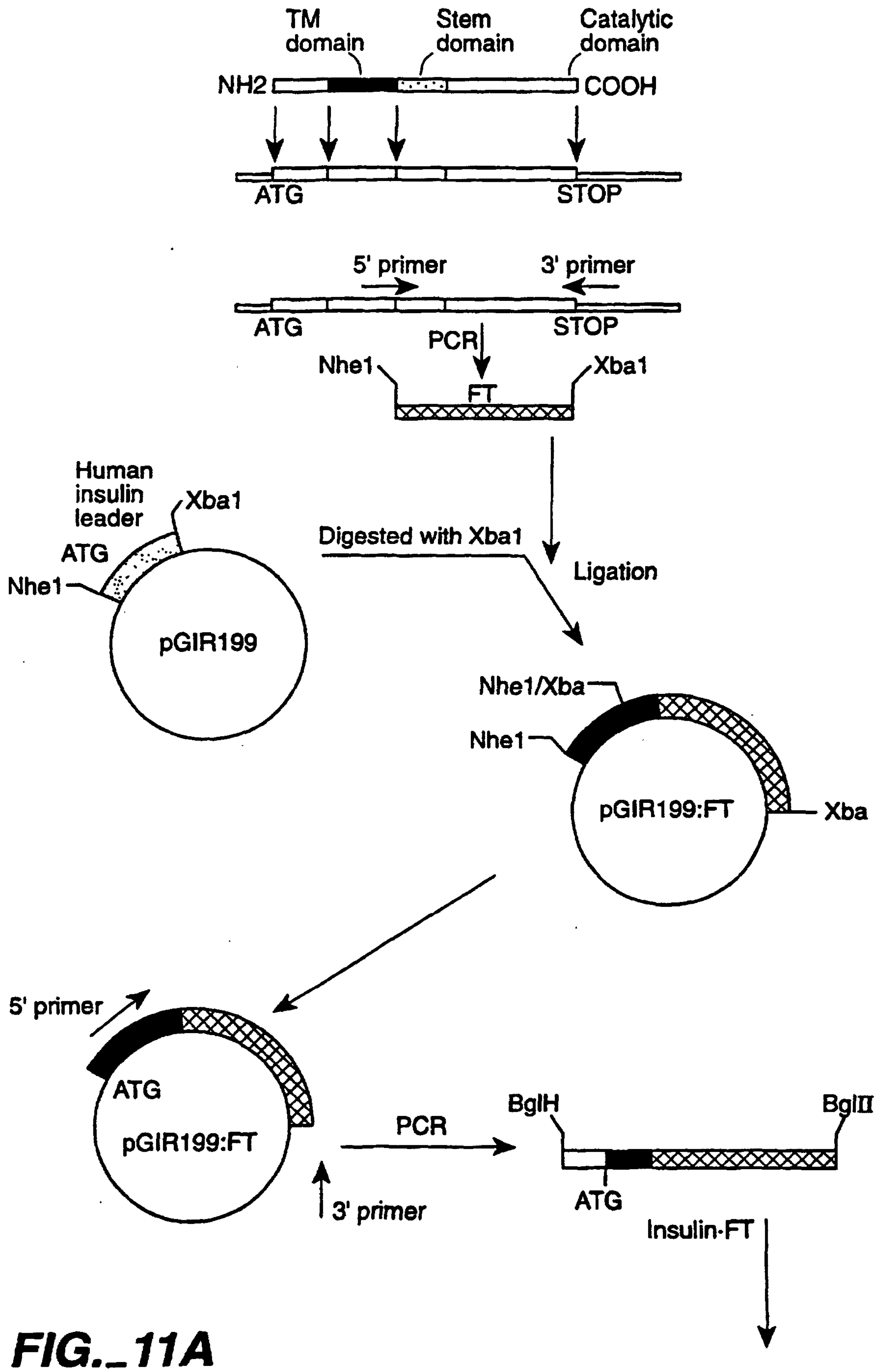
TM domain
Stem domain
Catalytic domain
NH2
COOH
ATG
STOP
5' primer
3' primer
ATG
STOP
PCR
Nhe1
Xba1
FT
Human insulin leader
Xba1
ATG
Nhe1
pGIR199
Digested with Xba1
Ligation
Nhe1/Xba
Nhe1
pGIR199:FT
Xba
5' primer
ATG
pGIR199:FT
3' primer
PCR
BglH
BglII
ATG
Insulin-FT

FIG._11A

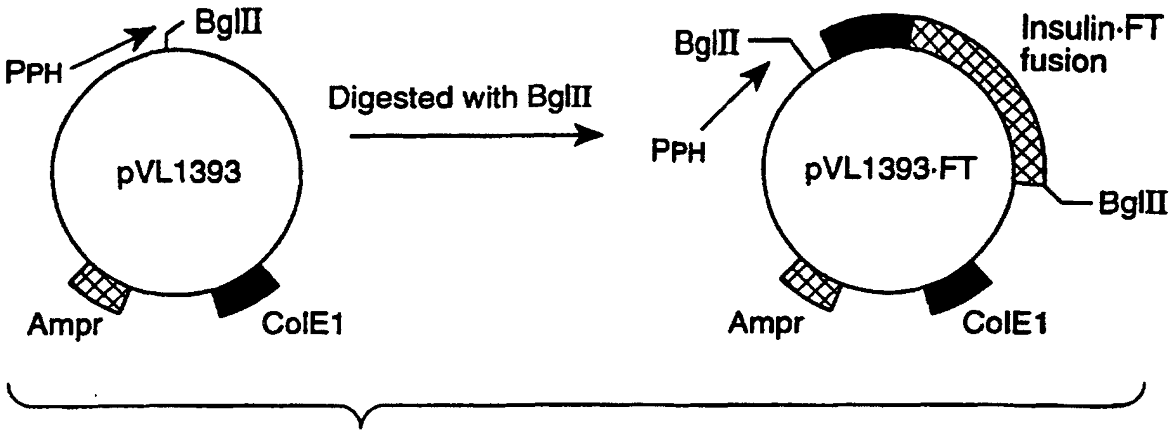


FIG._11B

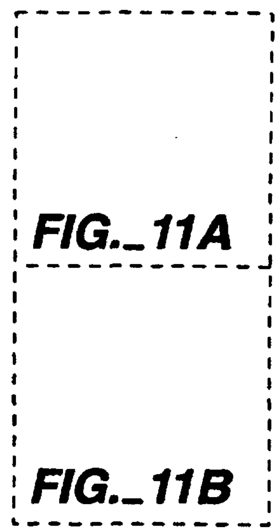


FIG._11